# EUROPÄISCHE PATENTSCHRIFT

(11) **EP 0 964 659 B1**
(45) Veröffentlichungstag und Bekanntmachung des Hinweises auf die Patenterteilung: **08.09.2004**
(21) Anmeldenummer: 98909280.4
(22) Anmeldetag: 24.01.1998
(51) Int. Cl.: A61F 2/06, A61F 2/01, A61B 17/12

(54) **STENT ZUR BEHANDLUNG PATHOLOGISCHER KÖRPERGEFÄSSE**
STENT FOR TREATING PATHOLOGICAL BODY VESSELS
EXTENSEUR POUR LE TRAITEMENT DE VAISSEAUX PATHOLOGIQUES

(30) Priorität: 31.01.1997 DE 19703482
(43) Veröffentlichungstag der Anmeldung: 22.12.1999
(73) Patentinhaber: Strecker, Ernst Peter, Dr.-med.Prof., D-76228 Karlsruhe (DE)
(72) Erfinder: Strecker, Ernst Peter, Dr.-med.Prof., D-76228 Karlsruhe (DE)
(74) Vertreter: Geitz, Holger
(86) Internationale Anmeldenummer: PCT/DE1998/000226
(87) Internationale Veröffentlichungsnummer: WO 1998/033454

(56) Entgegenhaltungen:
- WO-A-90/04982
- WO-A-92/05829
- WO-A-94/00179
- WO-A-94/03127
- WO-A-94/06502
- WO-A-94/06503
- WO-A-94/20044
- WO-A-95/18585
- FR-A- 2 678 508
- US-A- 4 300 244
- US-A- 5 116 365
- US-A- 5 304 194

## Beschreibung

Die Erfindung betrifft einen Stent zur Behandlung pathologischer Körpergefäße, der dauerhaft implantierbar ist und erst am Implantationsort während der Implantation seine bestimmungsgemäβe Form einnimmt, und mehrere Filamente umfasst, wobei die Filamente (2, 2') im bestimmungsgemäßen Zustand des Stents (1) an wenigstens einer im Bereich des Außenumfangs des Stents (1) liegenden Verbindungsstelle (4, 5) fest miteinander verbunden sind, wobei wenigstens zwei der Filamente (2, 2') in ihrem bestimmungsgemäßen Zustand über wenigstens einen Teil der Längserstreckung des Stents (1) in der Form zueinander gegenläufiger Wendeln (3, 3') vorliegen und diese Filamente (2, 2') der zueinander gegenläufigen Wendeln (3, 3') aus einem einzigen Filamentdraht bestehen, der an der Verbindungsstelle der beiden Wendeln (3, 3') einen Knick oder eine Schlaufe (4) zur Ausbildung einer gegenläufigen Wendel (3, 3') aufweist,

Ein solcher Stent ist aus der WO-A-94 20044 vorbekannt. Dabei besteht die vorbekannte Lösung darin, hinter einen Stent mit dem einen Drehsinn einen weiterer Stent mit einem anderen Drehsinn zu schalten, die demnach gegenläufig gewunden sind und über eine Verbindungsstelle miteinander verbunden sind.

Durch den Wechsel des Drehsinns soll eine leichtere Implantation und eine flexiblere Wandung erreicht werden.

Aus der WO-A-94 03127 ist ein weiterer Stent bekannt, der aus ebenfalls aus mehreren miteinander verbunden Filamenten aufgebaut ist, die zu einem Gestricke derart verbunden sind das sich geschlossene Ringe ausbilden die durch Verbindungsfilamente in Richtung der Längsachse des Stents miteinander verbunden sind. Allerdings bedingt die erwähnte Struktur des Stents , auch eine erschwerte Implantation, da der Stent in einer zusammengedrückten aber bereits die endgültige Struktur aufweisenden Aufbau durch einen Katheter in komprimierter Form zu implantieren ist. Außerdem fehlt dem vorbekannten Stent in seiner bestimmungsgemäß aufgeweiteten Form, die an sich vorteilhafte Spiralstruktur.

Es ist ferner grundsätzlich bekannt zur Behandlung pathologischer Körper- und Blutgefäße, Spiralstents aus Metall oder Kunststoff in ein erkranktes Körpergefäß zu einzuführen.

Derartige Behandlungen kommen bei krankhaften Gefäßverschlüssen oder Aneurysmen, insbesondere der Aorta, in Betracht. Die Implantation solcher Gefäßprothesen ist infolge des beträchtlichen Durchmessers dieser Prothesen erschwert. Meist ist nur eine chirurgische Implantation der Gefäßprothesen in Verbindung mit einer Öffnung des Gefäßes und einem anschließenden Gefäßverschluß mittels einer Gefäßnaht möglich. Im Falle der Behandlung eines Aorten-Aneurysmas werden Stents über die Beckenaterie eingeführt. Diese Behandlung wird durch in Verbindung mit Aorta-Aneurysmen gehäuft auftretende Stenosen und durch den geschlängelten Verlauf der Beckenaterien erschwert oder gänzlich vereitelt.

In den genannten Fällen, aber auch im Falle der Behandlung kleinerer Gefäße, wie etwa intrakranieller Gefäße, ist es vorteilhaft, Stents zu verwenden, die von einem kleinen Durchmesser zur Implantation auf einen größeren Durchmesser am Implantationsort aufgeweitet werden können. Demgemäß ist vorgesehen, ballonaufweitbare und selbstaufweitende Stents mit einem geeigneten Katheter in die zu behandelnden Gefäße zu implantieren. Jedoch erfüllen die genannten Stents bis jetzt noch nicht die technischen Voraussetzungen für einen störungsfreien Einsatz.

So handelt es sich beispielsweise bei dem sogenannten IN-Stent um eine elastische Spirale, die beim Einführen in das Körpergefäß vom Katheter auf einem kleineren Durchmesser gehalten, am Implantationsort mittels eines speziellen Mechanismus vom Katheter gelöst wird und sich sodann auf ihren Gebrauchsdurchmesser erweitert.

Nachteilig ist dabei, daß der Durchmesser des Spiralstents im erweiterten Zustand höchstens doppelt so groß ist wie im Einführzustand, wodurch relativ große Punktionsöffnungen zum Einführen dieser Art von Stent notwendig sind.

In diesem Zusammenhang ist in dem Aufsatz "Transluminally-placed coilspring endaterial tube grafts", Invest. Radiol. (1969) Nr. 4, Seiten 329 ff. von Charles Dotter die Verwendung eines Thermo-Memory-Drahtes bereits beschrieben worden.

Thermo-Memory-Drähte sind meist Nitinol-Drähte, also Nickel-Titan-Legierungen, die bei Temperaturen zwischen 400° und 500° Celsius in eine vorbestimmte Form gebracht werden und diese Form bis zu einer bestimmten Umwandlungstemperatur unterhalb der Körpertemperatur bewahren.

Unter der "Thermo-Memory-Eigenschaft" versteht man, daß diese Drähte bei einer entsprechend weiteren Abkühlung, beispielsweise mittels Eiswasser, ihre bisherige Form und Elastizität verlieren und anschließend als langgestreckter Draht freibeweglich und biegsam sind. Sobald der Draht wieder auf eine ungefähr der Körpertemperatur entsprechende Temperatur erwärmt wird, springt ein solcher Draht vollelastisch in die während der Wärmebehandlung eingeprägte Raumform zurück.

Charles Dotter schlägt vor, einen Wendelstent aus gekühltem Thermo-Memory-Draht in Form eines Ianggestreckten Drahtes zu implantieren, der anschließend aufgrund seiner beschriebenen Thermo-Memory-Eigenschaft am Implantationsort in die gewünschte Wendel- und Prothesenform springt.

Es hat sich gezeigt, daß derartig einfach gewendelte Stents in ihem bestimmungsgemäßen Zustand eine zu geringe Stabilität aufweisen und zudem schwierig einzuführen und exakt zu plazieren sind.

Aus der WO 94/03127 ist ein Stent bekannt, bei dem mehrrere in einer Einführungsform langgestreckte Drahtfilamente in ihrem bestimmungsgemäßen Zustand eine sich an die Gefäßwand anschmiegende Wellenform annehmen, wobei die Wellenlinien von jeweils zwei Filamenten derart gebildet sind, daß ein aus ungefähr ovalen Elementen zusammengesetztes Netzwerk entsteht. Die Stabilität dieses Netzwerks kann weiter dadurch erhöht werden, daß einander gegenüberliegende Wellenlinien an den Stellen wo sie sich am nächsten kommen, miteinander verbunden sind.

Nachteilig ist bei diesem netzwerkartigen Stent, daß die Implantation einer derart komplizierten Struktur insbesondere bei stark gekrümmten Gefäßen zu erheblichen Schwierigkeiten führt. Darüber hinaus erfordert der Aufbau eines solchen Stents aus einer Vielzahl einzelner Filamente einen Katheter mit einem relativ weiten Einführdurchmesser. Desweiteren gibt es bei diesem vorbeschriebenen Stent nur jeweils einen einzigen vorbestimmten Durchmesser im aufgeweiteten Zustand. Es ist daher schwierig, den Stentdurchmesser auf den Durchmesser der zu behandelnden Arterie abzustimmen.

Aus der WO 95/18585 ist ein Stent bekannt, bei dem die Filamente in der Form von in gleichem Drehsinn verlaufenden spiralförmigen Wendeln ausgebildet sind. Zur Schaffung von Bereichen mit unterschiedlicher Steifigkeit sind die Wendeln in Teilbereichen entlang der Längsrichtung des Stents mit unterschiedlicher Steigung ausgebildet. Derartige Stents sind jedoch relativ instabil und zeichnen sich durch eine schwierige Handhabung aus, weil der Stent beim Implantieren zum Verklemmen innerhalb des pathologischen Körpergefäßes neigt.

In der US-PS 5,116,365 ist ein ballonaufdehnbarer Stent offenbart, dessen Filamente ersichtlich nicht dauerelastisch ausgebildet sind. Die Filamente sind in Längserstreckung des Stents in der Form zickzackartiger, miteinander verschlaufter Bänder ausgebildet, die zur Schaffung der erforderlichen Elastizität in sich schlangenlinienförmig gewendelt sind. Dadurch entsteht eine mit etwa rautenförmigen Elementen zusammengesetzte Netzstruktur. Derartige Stents sind schwierig in der Handhabung.

Aus der WO 94/00179 ist ein Stent bekannt, bei dem die Filamente in Längserstreckung des Stents in der Form zickzackartig ausgebildeter Schlaufenstrukturen angeordnet sind.

Die spezielle Anordnung und Ausbildung der Filamentstrukturen ermöglicht zwar eine höhere Stabilität des implantierten, seine bestimmungsgemäße Form einnehmenden Stents, jedoch ermöglichen derart ausgebildete Stents nur jeweils einen einzigen vorbestimmten Durchmesser im aufgeweiteten Zustand. Bei Körpergefäβen mit demgegenüber insgesamt oder partiell kleineren Gefäßdurchmessern kann eine nicht unerhebliche Beeinträchtigung des Blutflusses einhergehen.

Aus der WO 92/05829 geht ein als Geflecht ausgebildeter Stent hervor, der nur temporär einsetzbar ist und deshalb nach einer gewissen Verweildauer wieder entfernt werden muß. Dieser Stent ist mit drei oder mehr einzelnen langgestreckten Filamenten ausgebildet, die im implantierten Zustand in Längserstreckung des Stents zueinander gegenläufige Wendelstrukturen ausbilden. Bei diesem Stent liegen die beiderseitigen Enden der den Stent bildenden Filamente konstruktionsbedingt im Bereich der zentralen Längsachse des Stents und sind dort mit einer flexiblen Hülse auf einem stab- bzw. rohrförmigen innen liegenden Katheter fixiert. Dadurch kann der Blutfluß erheblich beeinflußt werden.

Aufgabe der vorliegenden Erfindung ist es daher, einen Stent der vorgenannten Art und Zweckbestimmung zu schaffen, der sich durch eine hohe Stabilität sowie eine einfache Handhabung bei der Implantation auszeichnet und der darüber hinaus eine hohe Expansionsrate, d.h. ein besonders hohes Verhältnis des Stentdurchmessers im expandierten Zustand zum Stentdurchmesser im Einführungszustand aufweist.

Gelöst ist diese Aufgabe bei einem Stent mit den Merkmalen des Oberbegriffs des Patentanspruchs 1 durch die Merkmale des kennzeichnenden Teils des Anspruchs 1.

Der erfindungsgemäße Stent besteht also in seinem bestimmungsgemäßen Zustand aus wenigstens zwei Wendeln, die gegeneinander, also im entgegengesetzten Drehsinn, angeordnet sind, und hat die äußere Form eines Tubus.

Eingeführt wird ein solcher Doppelwendel-Stent mit den Filamenten im gestreckten Zustand, also mit einer nahezu eindimensionalen Struktur.

Ein derart ausgebildeter Stent weist eine hohe Stabilität bei gleichzeitiger hoher Flexibilität auf. Die Steigungen der einzelnen Wendelschlaufen sind dabei über die Gesamtlänge des Stents stark veränderbar. Dies ermöglicht insbesondere eine Verlegung des Stents in stark gekrümmten Körpergefäßen, ohne daß dabei eine Beeinträchtigung der Stabilität des Stents oder eine Verkleinerung des Lumens des Körpergefäßes, auch nur abschnittsweise, in Kauf genommen werden müßte. Durch die Variierbarkeit der Steigungen der einzelnen Wendeln ist auch die Gesamtlänge des Stentkörpers veränderlich. Hierdurch kann beispielsweise eine bessere Verankerung des Stents innerhalb des Körpergefäßes erreicht werden. Weiterhin ist eine dem Gefäß angepaßte jeweils unterschiedliche Tragfähigkeit bzw. Stützfähigkeit des Stents bei der Behandlung von Gefäßkrankheiten, wie zum Beispiel Aneurysmen, erzielbar. So werden etwa an den Enden eines Aneurysma-Stents Wendelschlaufen mit geringer Steigung, also hoher Dichte, benötigt, um den Stent hier zu verankern, während im Bereich des Aneurysmas selbst weniger Wendelschlaufen benötigt werden.

Eine besonders schonende und einfache Implantation des Stents ist insbesondere dann möglich, wenn das Filament aus einem Thermo-Memory-Draht hergestellt ist. In diesem Zusammenhang ist insbesondere die Verwendung von Nitinol-Drähten empfehlenswert. Es sind jedoch auch Kunststofffilamente mit geeigneten Thermo-Memory-Eigenschaften einsetzbar. Anstelle des aus Thermo-Memory-Draht gefertigten Filaments können durchaus auch hoch- bis superelastische Drähte als Filamente gewählt werden, die infolge ihrer besonderen elastischen Eigenschaften am Implantationsort in ihre bestimmungsgemäßen Wendelformen gelangen. Ein derartiges Filament kann ebenfalls aus Nitinol, aus Edelstahl oder auch aus geeigneten Kunststoffen gefertigt sein.

Erfindungsgemäß bestehen zwei jeweils eine Wendel bildende Filamente aus einem einzigen Filamentdraht, der etwa am distalen Ende des Stents einen Knick, einen Bogen oder eine Schlaufe aufweist, derart, daß die Ausbildung zweier gegenläufiger Wendeln ermöglicht wird. Eine solche Ausführung weist eine hohe Stabilität auf.

Anstatt die Doppelwendelstruktur des erfindungsgemäßen Stents aus einem Filamentdraht herzustellen, der entsprechend in Form gegenläufiger Wendeln gebogen wird,ist es auch möglich, die Doppelwendelstruktur des Stents aus einem rohrförmigen Werkstück herauszuschneiden. Das Herausschneiden kann sehr effizient mit Hilfe eines Lasers erfolgen. Der besondere Vorteil dieser Ausgestaltung liegt darin, daß die gegenläufigen Wendeln an ihren Überkreuzungspunkten bereits miteinander verbunden sind, wodurch sich etwaige zusätzliche Verbindungsmittel erübrigen.

Eine ebenfalls stabilisierende Wirkung des Stents wird erreicht, indem zwei aus Einzelfilamenten aufgebaute Wendeln etwa am distalen Ende des Stents miteinander verbunden sind. Diese Verbingung kann etwa durch Verkleben, Verlöten oder Verschweißen der beiden Stentdrähte hergestellt sein, oder aber es kann sich dabei um eine Muffe handeln, die beide Stentdrähte übergreift und somit eine Verbindung herstellt, die eine begrenzte axiale Verschiebbarkeit der Stentdrähte zuläßt, eine Verdrehung der Stentdrähte gegeneinander jedoch verhindert. Auf diese Weise wird auch die Flexibilität des Stents verbessert. Eine entsprechende Verbindung kann auch am proximalen Ende des Stents bestehen.

Diese Verbindungspunkte zweier Wendeln befinden sich auf dem Außenumfang, also auf dem Mantel des tubusförmigen Stents. Auf diese Weise wird eine Störung des durch das Körpergefäß hindurchströmenden Blutflusses so gering wie möglich gehalten. Vorzugsweise sind die Verbindungsstellen radial nach außen aufgebogen, damit diese auf keinen Fall in das Lumen hineinragen. Insbesondere bei einem kurvigen Verlauf des Gefäβes passen sich bei einer solchen Ausführungsform die Stentenden dem Kurvenverlauf des Körpergefäßes an.

Um die Stabilität des Stents weiter zu erhöhen, sind die gegenläufigen Wendeln zumindest teilweise an den Punkten, an denen sich die Wendeln überkreuzen, miteinander verbunden. Eine besonders vorteilhafte Verbindung erfolgt nach Anspruch 8 durch Fäden guter biologischer Verträglichkeit, etwa Nylonfäden, die mit einer der Wendeln verbunden sind, und die an vorgegebenen Stellen Schlaufen aufweisen, durch die die jeweils andere Wendel hindurchgeführt ist. Eine solche Ausgestaltung ermöglicht die problemlose Entfaltung der Doppelwendel während der Implantation. Um eine Reibung zwischen den Wendeln oder der Wendel am Gewebe zu vermeiden, die zur Abnutzung der Filamente, bzw. zu Gewebsreizungen führen könnte, sollten die Wendelschlaufen dabei derart fest miteinander verbunden sein, daß im bestimmungsgemäßen Zustand die Möglichkeit des Bewegens der Wendeln gegeneinander auf ein Minimum reduziert wird.

Bei der Ausführungsform nach Anspruch 9 besitzt der Stent drei oder mehr Filamente. Während zwei der Filamente in der oben beschriebenen Weise in ihrer bestimmungsgemäßen Form als gegenläufige Wendeln mit vorzugsweise gleicher Steigung im Stent vorliegen, verlaufen die übrigen ebenfalls wendelförmig ausgebildeten Filamente mit einer anderen Steigung als die beiden gegenläufigen Wendeln. Dadurch verläuft das dritte bzw. verlaufen die folgenden Filamente zumindest teilweise in den von den gegenläufigen Doppelwendeln ausgesparten Lücken. Insgesamt wird damit die Stabilität des Stents weiter erhöht und darüber hinaus ein über die Gesamtlänge des Stents überwiegend gleichbleibendes Lumen gewährleistet.

Zwar ist der Aufbau des Stents aus zwei gegenläufigen Wendeln von hoher Stabilität und Flexibilität, dennoch kann es fallweise vorteilhaft sein, wenn der Stent über einen Teil seiner Gesamtlänge aus einer Doppelspirale besteht, die nicht gegenläufig, sondern gleichsinnig gewendelt ist. In diesem Bereich bestehen somit keine Überkreuzungen der Filamente miteinander. Zudem besteht abschnittsweise im Stent eine noch weiter erhöhte Flexibiltät, was beispielsweise bei der Implantation in stark gekrümmten Gefäßen von Vorteil ist.

Bei der Ausführung nach Anspruch 10 ergibt sich eine verbesserte Federwirkung in Längsrichtung des Stents. Ein solcher Stent ist nicht nur von höherer Tragfähigkeit und Stützfähigkeit, sonder er weist auch eine höhere Körperverträglichkeit, insbesondere bei einer Implantation im Kurven- oder Beugebereich von Gefäßen auf

Eine vorteilhafte Weiterbildung der Ausführungsform nach Anspruch 10 ergibt sich, wenn jeweils zwei Filamente wenigstens über einen Teil der Längserstreckung des Stents jeweils gleichartige, zueiander gegenläufige bogenförmige Abschnitte aufweisen.

Zweckmäßigerweise sind einander gegenläufige bogenförmige Abschnitte zweier Filamente miteinander verbunden. Dabei können die bogenförmigen Abschnitte in der Art eines Maschendrahtzaunes miteinander verhakt sein, wodurch eine höhere Flexibiltät und Stabilität des Stents erreicht wird. Eine besonders flexible, aber dennoch stabile Verbindung erfolgt durch Textilfäden, die fest mit dem einen Filament verbunden sind und.das jeweils andere Filament aufnehmende Schlaufen aufweisen. Eine solche Ausführungsform erleichtert insbesondere die Handhabbarkeit beim Einführen des Stents in Form langgestreckter Filamente. Zugleich ist bei einer solchen Verbindung eine axiale Beweglichkeit der Filamente gegeneinander gewährleistet, was wiederum im Bereich stark 15 gekrümmter Körpergefäße von Vorteil ist. Alternativ zur flexiblen Verbindung der Bögen untereinander ist jedoch auch eine feste Verbindung möglich, etwa mittels Muffen, die die beiden Bögen übergreifen oder aber durch Schweiβen, Löten oder Kleben. Auch eine Kombination von Muffen und den anderen genannten Befestigungsmöglichkeiten ist vorstellbar. Im Falle einer festen Verbindung der Bögen miteinander kann der Einführradius durch den Katheter besonders klein gehalten werden.

Eine besonders vorteilhafte Ausführungsform ist nach Anspruch 19 gegeben. Danach weisen die Filamente des Stents eine alternierende Form von Bögen und Wendeln auf und sind derart gegeneinander versetzt, daß jeweils ein Bogen mit einer Wendelschleife kreuzt. Hierdurch entsteht eine nahezu rechtwinklige Überkreuzung, wodurch die Stabilität des Stents weiter verbessert wird. Vorzugsweise weist einer der Filamente am bestimmungsgemäßen Kreuzungspunkt eine kleine Ausbuchtung auf, in der das korrespondierende Filament aufnehmbar ist. In ihrem bestimmungsgemäßen Zustand rasten die entsprechenden Filamente an dieser Stelle ein, was zu einer noch weiteren Erhöhung der Stabilität führt.

Vorzugsweise ist der Stent in seinem bestimmungsgemäßen Zustand im Querschnitt den Körpergefäßen, für die er vorgesehen ist, angepaßt. So kann es vorteilhaft sein, zumindest abschnittsweise ein ovales oder elliptisches Lumen des Stents vorzusehen. Ein weiteres Lumen wird zum Beispiel für den proximalen Anteil des Stents in der A. carotis communis oder im Bulbus der A. carotis interna benötigt, während das distale Ende einen geringeren Durchmesser haben muß, da sich der Arterienquerschnitt im allgemeinen hier verjüngt.

In weiterer bevorzugter Ausgestaltung wird der Stent als Doppelstent verwendet. Dabei besitzt nur ein Abschnitt des Stents eine einzelene tubuläre Form nach der vorher beschriebenen Art. In einem zweiten Abschnitt weist der Stent dagegen zwei Lumina auf, die jeweils durch wenigstens ein wendelförmiges Filament gestützt sind und die einander teiweise berühren. Somit bildet sich über wenigstens einen Teil der Längserstreckung des Stents ein Doppelstent aus. Die Lumina der beiden Nebenstents können dabei, wie oben beschrieben, oval ausgebildet sein, oder aber die Querschnitte zweier zueinander gespiegelter "D" aufweisen. An ihren Berührungsstellen können die beiden Nebenstents auch nach der Art, wie vorher beschrieben, über Fäden miteinander verbunden sein, welche an wenigstens einer der Filamente befestigt sind und die Schlaufen aufweisen, in denen das jeweils andere Filament aufgenommen ist. Es ist auch weiterhin möglich, daß die beiden Wendeln des Doppelstents aus Filamenten bestehen, die so angeordnet sind, daß die Schlaufen, in ihrem Querschnitt gesehen, die Form einer Acht aufweisen, sich also überkreuzen. Bei einer solchen Ausführungsform erübrigt sich eine Befestigung der beiden Nebenstents miteinander.

Die Stents mit den vorbeschriebenen Merkmalen sind nach Ansprüchen 18 mit einer verformbaren Membranumhüllung an der Außen- oder Innenseite der Doppelwendelstruktur bezogen. Die Membran ist dabei jeweils an den Enden des Stents befestigt, und steht im bestimmungsgemäßen, das heißt aufgedehnten Zustand des Stents nicht unter Längszugbelastung. Wird der Stent in die Länge gezogen, ist die Membran entsprechend streckbar. Als vorteilhaftes Material für eine derartige Membran kommen beispielsweise hochelastischer Kunststoff, Silikon oder Latex in Frage. Alternativ zu elastisch verformbaren Membanumhüllungen kann jedoch ein Gewirke oder ein Gestricke eingesetzt werden, dessen Maschen bei der Implantation des Stents von einer Einführungsform, in der die Fäden des textilen Gewebes im wesentlichen parallel zur Stentachse verlaufend in eine Aufweitungsform, in der die die maschenbildenden Fäden im wesentlichen senkrecht zueinander stehen, überführbar sind. Auch können die Fäden eines solchen Gewirkes texturiert sein, d.h. eine dehnbare, spiralförmige Struktur aufweisen.

Besonders vorteilhaft ist die Verwendung von Textilmaterial, wie zum Beispiel elastisches Gewebe oder Polytetrafluoräthylen (PTFE), das sich ebenfalls entsprechend strecken läßt. In diesem Falle würden die offenen Maschen des Gewebes schnell durch Thrombenbildung geschlossen, so daß sich auch in diesem Falle eine geschlossene Wand bildet.

Um zu verhindern, daß sich das Textilgewebe im aufgedehnten Zustand des Stents zwischen den Stentfilamenten in den Innenbereich des Stents eindringt, sind nach Anspruch 20 in die Gewebestruktur der textilen Membranumhüllung vorzugsweise sich kreuzende Metallfäden eingewirkt, die das Hineinfallen des Textils in den Bereich zwischen den Stentwendeln verhindern.

Eine ebenfalls vorteilhafte Möglichkeit, einen Stent mit einer Umhüllung zu versehen, erfolgt gemäß den Merkmalen der Ansprüche 21 bis 33.

Bei der Weiterbildung nach Anspruch 27 hat der Stent die Form eines Drahtschlaufenskeletts, dessen einzelne Doppelwendelschlaufen durch mit den Filamenten verbundene Gewebestrukturen oder Fasern gegenüber der Gefäßwand abgeschirmt sind. Der Stentkörper wird somit nur zum Teil durch das Filament gebildet und besteht zum anderen Teil aus Fasern bzw. Gewebeabschnitten.

Durch die Abschirmung der Filamente gegenüber der Gefäßwandung mittels der Gewebestrukturen/oder, Fasern ist die Körperverträglichkeit des Stents erheblich verbessert. Ein solcher Stent entspricht also einem Stentgraft. Ein entscheidender Vorteil der Weiterbildung gemäß Anspruch 27 liegt jedoch auch darin, daß im Unterschied zu den bisher bekannten Drahtstents mit aneinander anliegenden Drahtschlaufen im Bereich der Gewebe- und Faserabschnitte die Wandungen des Stentkörpers Membraneigenschaften, also beispielsweise eine Diffusionsfähigkeit aufweisen. Die Gefäßwandungen können damit zunächst im Wege der Diffusion weiterversorgt werden. Zusätzlich ist eine etwaige lokale Medikamentenabgabe durch eine Beschichtung der neuartigen Stentwandung möglich. Hierdurch ist die Gefahr einer etwaigen Intima-Hyperplasie oder einer sonstigen neplastischen Wucherung der zu behandelnden Gefäßwandungen reduziert. Ein weiterer Vorteil ist darin zu sehen, daß sich eine verstärkte Anlagerung von, Bindegewebszellen bzw. eine verstärkte Thrombosierung im Bereich der Gewebe- oder Faserabschnitte der Stentwandung ergibt. Im Unterschied zu der bekannten Embolisationswendel zum Verschließen von Gefäßen nach Gianturco bildet der erfindungsgemäße Stent gewissermaßen ein mit Fasern versehenes Rohr, das das Gefäßvolumen offenläßt. Aufgrund der beschriebenen Zellanlagerungen bildet sich nach und nach infolge der Thrombogenität der Gewebestruktur bzw. der Fasern eine biologische Wand. Schließlich ist es möglich, die Fasern derart zu präparieren, daß sie nach erfolgter Implantation Medikamente, etwa zur Thrombenerzeugung abgeben können, um eine möglichst schnelle Abdichtung der Wandungen des Stentkörpers zu erreichen.

Besonders vorteilhaft ist es, wenn sich die von unterschiedlichen Wendeln und/oder Bogenabschnitten ausgehenden Gewebestrukturen und/oder Fasern mit ihren jeweils freien Enden zumindest teilweise berühren. Auf diese Weise ist das Drahtfilament von einem Mantel aus Fasern und/oder Gewebestrukturen umgeben. Aufgrund der beschriebenen Zellanlagerung begünstigt dies infolge der Thrombogenität der Gewebestruktur bzw. der Fasern die Ausbildung einer biologischen Wand. Hierdurch können beispielsweise Aneurysmen vom normalen Blutstrom abgesperrt werden, womit die Gefahr einer Ruptur des Aneurysmas wirksam unterbunden, zumindest aber erheblich gemindert ist. Die Behandlung von Aneurysmen ist insbesondere bei der Aorta abdominalis im infrarenalen Anteil, jedoch auch bei intrakraniellen kleineren Aneurysmen besonders wertvoll.

Der Stent kann ohne die Verwendung körperfremder Klebstoffe, die überdies von zweifelhafter Haltbarkeit und Körperverträglichkeit sind, derart hergestellt werden, daß wenigstens ein Filament unter Einschluß der Gewebestrukturen oder Fasern umhüllt und/oder umwickelt ist. Diese Umhüllung oder Umwicklung kann mittels eines anderen Textils oder Fadens erfolgen. Eine zusätzliche Befestigung der Gewebestrukturen oder Fasern ist nicht erforderlich.

In weiterer bevorzugter Ausgestaltung der Erfindung kann die Befestigung der Gewebestrukturen oder abstehenden Fasern einfach dadurch erfolgen, daß als Filament ein aus mehreren Filamenten verschlungenes Filament erzeugt wird, wobei innerhalb der Verschlingungen der einzelnen Filamente die Gewebestrukturen und Fasern gehalten sind. Die Gewebestrukturen und/oder Fasern sind also durch Öffnungen hindurchgeführt, die zwischen den miteinander verschlungenen Teilfilamenten vorhanden sind. Statt dessen ist es auch möglich, in einem Filament Öffnungen vorzusehen, durch die die Gewebestrukturen und/oder Fasern hindurchgezogen sind. Zumindest bei einer derartigen Ausführungsform weist das Filament zweckmäßigerweise einen rechtekkigen Querschnitt auf. Auch bei diesen Ausführungen ist die Verwendung zusätzlicher Kleber oder sonstiger Befestigungsmittel für die Gewebestrukturen oder Fasern entbehrlich.

Zweckmäßigerweise sind die sich von einem Filament radial forterstreckenden Gewebestrukturen und/oder Fasern abschnittsweise und/oder je nach radialer Richtung von unterschiedlicher Länge, entsprechend den Erfordernissen im jeweiligen Körpergefäß.

Bei einer besonders vorteilhaften Ausgestaltung, die in Patentanspruch 26 angegeben ist, sind die Gewebestrukturen und/oder die Fasern derart an den Filamenten befestigt, daß sich ihre freien Enden unter Ausbildung einer wellenlinienförmigen Begrenzungslinie zumindest annähernd berühren. Beim Übergang in den bestimmungsgemäßen Zustand wird damit eine besonders vorteilhafte Anpassung des Mantels aus Gewebestrukturen und/oder Fasern an die Wendelstrukturen gewährleistet.

Ein besonders dichter und fester Stentmantel wird dadurch hergestellt, daß sich die Gewebestrukturen und/oder die Fasern zwischen den einzelnen benachbarten, von den Filamenten gebildeten Wendeln und/oder Bogenabschnitten zumindest teiweise überlappen.

Gemäß den Ansprüchen 28 und 29 können die Gewebestrukturen des Stents zur Erzeugung diffusionsfähiger Membranabschnitte ebenso aus einer Textil- wie aus einer Metallstruktur hergestellt sein. Im Falle der Verwendung von Textilgeweben weisen die Membranabschnitte kleinere Poren und im Falle der Verwendung von Metallgeweben größere Poren auf. Bei kleinporigen Gewebeabschnitten erfolgt die Thromboisierung und Organisierung der eingebrachten Strukturen beschleunigt. Metallgewebestrukturen weisen hingegen eine höhere Querstabilität auf. Es kann jedoch auch eine Kombination von Metallgewebsstrukturen und Textilgewebsstrukturen sinnvoll sein.

Die Querstabilität eines derartigen Stents kann auch dadurch erhöht werden, daß die Gewebestrukturen zumindest abschnittsweise fransenartig eingeschnitten sind. Die Gewebestrukturen können sich bei entsprechender Länge der Fransen versteifend und dichtend überlappen und verzahnen.

Das gleiche Ziel kann auch dadurch erreicht werden, daß die Gewebestrukturen mit einem Haftmittel, vorzugsweise einem Klettverschluß, zur Verbindung der einander überlappenden Gewebestrukturen versehen werden. Die Verwendung entsprechender Klettbandverschlüsse stellt überdies sicher, daß die langgestreckten Filamente am Implantationsort ihre Wendelformen erreichen. Dieser Effekt der Haftung kann auch dadurch erreicht werden, daß am Filament dünne Gewebestreifen, die abwechselnd Häkchen und Schlaufen nach der Art von Klettverschlußstreifen aufweisen, angeordnet sind.

Dadurch, daß gemäß Patentanspruch 32 der Querschnitt der verwendeten Gewebestrukturen mit zunehmendem Abstand vom jeweiligen Filament nach außen hin abnimmt, ist sichergestellt, daß der Stent auch im Bereich der Überlappungen einen im wesentlichen gleichbleibenden Außendurchmesser aufweist. Zweckmäßigerweise besteht die Gewebestruktur und/oder bestehen die Fasern aus einem elastischen Material. Dadurch wird erreicht, daß die Fasern nach der Passage durch den Katheter sich selbsttätig zu ihrer radial vom Filament forterstreckenden Bestimmungslage ausrichten. Die Fasern können mittels einer maschinell fertigbaren Naht mit dem Filament verbunden sein. Dies ermöglicht eine besonders einfache Fertigung derartiger Stents.

Um den Stent in optimaler Weise gegenüber dem Körpergefäß abzudichten, ist es zweckmäßig, etwa in Verbindung mit der Behandlung von Aneurysmen, die Länge der Fasern so zu bemessen, daß das Geflecht dieser Fasern vorzugsweise in die entsprechende Gefäßaussackung hineinragt und hierdurch im Bereich der Gefäßaussackung eine verstärkte Thrombosierung stattfindet. Ebenso ist es denkbar, daß die Fasern bevorzugt radial in das Innere des Stents hineinragen, um eine Anastomose mit einem etwa darunterliegenden Stent zu bilden. Die Verbindung zu einem zweiten eingeschobenen Stent wird bei dieser Weiterbildung besser abgedichtet.

Die Implantation eines Stents und seine lagerichtige Plazierung kann dadurch erleichtert werden, daß wenigstens ein Filament mit speziellen Markierungen versehen ist, die etwa die Beobachtung des Stents in der Fernsehdurchleuchtung erleichtern. Es sind aber auch andere diagnostische Verfahren denkbar, wie z.B. Magnet-Resonanz-Tomographie oder Ultraschall.

Als Alternative zu den vorgenannten Ausführungsformen, bei denen der Innenbereich des Stents von einer Doppelwendelstruktur umschlossen wird, weist der Stent gemäß Anspruch 38 eine Doppelwendelstruktur auf, bei denen im wesentlichen parallel verlaufende Filament-Drähte in vorbestimmten Abständen entlang der Längserstreckung des Stents jeweils zueinander entgegengesetzt gewendelte Schlaufen ausbilden. Der Stentaußenradius wird von den Radien der Schlaufen bestimmt. Während der Implantation werden die Schlaufen in gestrecktem Zustand an den im übrigen langgestreckten Stentkörper angelegt. Am Implantationsort richten sich die vorzugsweise aus superelastischen oder Thermo-Memory-Material bestehenden Schlaufen zu ihrer Implantationsform auf. Wie die zuvor beschriebenen Ausbildungsformen kann auch dieser Stent von einer Membran aus elastischem Material umgeben und die Filamente des Stents können wiederum mittels Muffen miteinander verbunden sein.

Besonders vorteilhaft ist es, wenn die Implantation des Stents mit einer Implantationsvorrichtung derart erfolgt, daß zunächst ein Katheter in das zu behandelnde Körpergefaäß eingeschoben wird. Der Katheter ist so bemessen, daß sowohl der Stent, wie auch eine spezielle Pusher-Anordnung aus zwei Pushern in das Körpergefäß hineingeschoben werden kann. Die Pusher-Anordnung besteht dabei aus einem äußeren und einem inneren Pusher. Der äußere Pusher hat einen Durchmesser, der dem Innendurchmesser des Einführkatheters enspricht, und besitzt zugleich etwa den gleichen Außenumfang wie der Stent in seinem Einführungszustand, also etwa dem Gesamtumfang der eingeschobenen langgestreckten Filamente. Mit Hilfe dieses Pushers wird der Stent durch den Katheter hindurch bis an seinen bestimmungsgemäßen Ort in das Körpergefäß vorgeschoben, wo er seinen bestimmungsgemäßen Zustand, d.h. mit der Doppelwendelstruktur der Filamente, einnimmt. Durch den äußeren Pusher hindurch erstreckt sich axial eine Bohrung, durch die ein zweiter, innerer Pusher geschoben wird. Dieser dünne, jedoch aus einem festen Material, etwa Nitinol,gefertigte Pusher hat an seinem distalen Ende Mittel, mit denen er mit dem distalen Ende des Stents verbindbar ist.

Beim Einführen des Stents in seinem gestreckten Zustand in ein Körpergefäß wird dieser mit Hilfe des äußeren Pushers im Katheter vorgeschoben. Beim Heraustreten des Stents aus dem Katheter, d.h. wenn sich bereits einige Schlaufen der Doppelwendel im Gefäß gebildet haben, wird der Stent mit Hilfe des inneren Pushers koaxial im Gefäß gehalten. Ein Zurückspringen oder ein Vorspringen des Stents im Körpergefäß kann dadurch vermieden werden. Auf diese Weise ist eine exakte Plazierung des distalen Stentendes im Körpergefäß möglich. Das distale Stentende wird auch dann noch durch den dünnen Pusher an seinem Platz gehalten, wenn der Katheter zurückgezogen und/oder der Stent durch den äußeren Pusher vorgeschoben wird. Alternativ kann der Stent auch, nach Plazierung des Katheters, mittels des inneren Pushers, der mit der distalen Stentspitze verbunden ist, in das Körpergefäß hineingezogen werden. Dadurch wird der Stent während des Implantationsvorganges axial gestreckt, wodurch die Reibung zwischen Filamenten und Katheterwand verringert wird. Erst wenn der Stent vollständig in das Körpergefäß eingebracht worden ist, wird der dünne Pusher von der Stentspitze gelöst. Es ist prinzipiell möglich, den Stent mit Hilfe nur eines der Pusher im Körpergefäß zu plazieren; jedoch ermöglicht die Kombination beider Pusher eine besonders exakte und störungsfreie Implantation und Plazierung des Stents im Körpergefäß.

Als vorteilhaftes Verbindungsmittel zwischen innerem Pusher und Stent bietet sich ein Gewinde an, das vorzugsweise am distalen Ende des inneren Pushers angeordnet ist und das in entsprechende Verbindungsmittel des Stents, etwa eine von den beiden Wendeln der Doppelwendel am distalen Ende des Stents gebildeten Schlaufe, eingreift. Selbstverständlich kann auch der Stent an seinem distalen Ende entsprechende Verbindungsmittel aufweisen, etwa in Form einer dem Gewinde des Pushers angepaßten Gewindebohrung. Alternativ können auch einander entsprechende, an der Stentspitze und dem Pusher angeordnete Haken zum Einsatz kommen. Es ist auch möglich, den Stent und den Pusher durch eine spezielle Verlötung miteinander zu verbinden, die sich im Körper durch einen entsprechenden elektrischen Strom auflösen läßt. Ein solches Prinzip wird bereits bei Embolisationsspiralen angewendet.

Bei einer Reihe von Behandlungen hat sich herausgestellt, daß das Lumen eines Körpergefäßes nach einiger Zeit auch ohne die Unterstützung eines Stents offenbleibt. Es ist somit zweckmäßig, Vorrichtungen vorzusehen, mit deren Hilfe der Stent nach einiger Zeit aus dem Gefäß wieder entfernbar ist. Aus diesem Grunde ist der Stent mit dem äußeren Pusher lösbar verbunden. Als Verbindungsmittel kommt wiederum eine Gewindeverschraubung oder aber eine am proximalen Ende des Stents angeordnete Haltelasche, die mit einem entsprechenden Haken des Pushers gefaßt werden kann, in Frage. Es ist somit nicht notwendig, den Pusher während der gesamten Stentliegezeit im Körper verweilen zu lassen. Nach einer vorbestimmten Zeit kann der Stent aus dem Körpergefäß am äußeren Pusher durch den Katheter hindurch herausgezogen werden.

Bestehen die Filamente des Stents aus einem Material mit Thermo-Memory-Eigenschaften, sollte dieser durch einen mit Kühlflüssigkeit gefüllten Katheter implantiert werden, um zu vermeiden, daß der Stentdraht schon innerhalb des Katheters in die voluminösere Wendelform springt und dann bedingt durch die Reibung zwischen dem Stentdraht und der Innenwandung des Katheters die Implantation behindert.

Als Alternative zu der zwei Pusher aufweisenden Implantationsvorrichtung der vorgenannten Art bietet sich zur Implantation des erfindungsgemäßen Stents-eine Implantationsvorrichtung an, bei der ein mittels eines Katheters in das Körpergefäß einführbarer Pusher an seinem distalen Ende mit einem Stützmittel zum Abstützen des Stents im Innern des Katheters versehen ist.

Als besonders vorteilhaftes Stützmittel ist eine am distalen Ende des Pushers angeordnete Gabel vorgesehen, die während der Implantation die Filamente an ihren Überkreuzungspunkten oder Verbindungen bzw. an den die Filamente miteinander verbindenden Muffen mit ihren Zinken übergreift und somit eine besonders sichere Führung des Stents bei der lagerichtigen Implantierung im Körpergefäß ermöglichen.

In einer anderen vorteilhaften Weiterbildung ist als proximales Stützmittel eine Zangenvorrichtung vorgesehen, mittels der die Filamente oder die diese miteinander verbindenden Muffen beim Implantieren des Stents fest umgriffen, jedoch, etwa nach einer axialen Betätigung des Katheters, von diesem gelöst werden. Auf diese Weise kann der Stent bei einer etwaigen Fehlplazierung auch in Richtung des Katheters zurückgezogen werden.

In einer anderen vorteilhaften Weiterbildung ist das Stützmittel des Pushers hakenförmig ausgebildet und der Stent weist an seinem distalen Ende eine die Filamente miteinander verbindende Muffe auf, in die eine Kerbe eingeprägt ist, die mit dem Haken formschlüssig und innerhalb des Katheters axialfest verbindbar ist. Auch diese Ausführungsform zeichnet sich durch eine besonders zuverlässige Führung des Stents während der Implantation aus.

Nachstehend wird die Erfindung anhand mehrerer in der beigefügten Zeichnung schematisch dargestellter Ausführungsbeispiele erläutert. In nicht maßstäblichen Ansichten zeigen:
- Fig. 1: einen Stent mit zwei gegenläufig gewendelten Filamenten in seinem bestimmungsgemäßen Zustand in einer Draufsicht,
- Fig. 2: den Stent in einer Ansicht gemäß Pfeil II in Fig. 1,
- Fig. 3: den Stent in einer Ansicht gemäß Pfeil III in Fig. 1,
- Fig. 4: einen aus drei wendelförmigen Filamenten bestehenden Stent in einer Ansicht ähnlich Fig. 2,,
- Fig. 5: einen Stent teilweise in seiner langgestreckten Implantationsform in einem Katheter und teilweise in seinem bestimmungsgemäßen Zustand in einem Körpergefäß,
- Fig. 6: im Ausschnitt VI aus Fig. 5 einen vergrößerten Überkreuzungsbereich zweier Filamente,
- Fig. 7: einen anderen Stent im implantierten Zustand,
- Fig. 8: einen Stent mit bogenförmigen Filamentabschnitten in seinem bestimmungsgemäßen Zustand, wobei die Filamentabschnitte durch Verbindungsmittel miteinander verbunden sind,
- Fig. 9: einen Stent in seinem bestimmungsgemäßen Zustand mit bogenförmigen Filamentabschnitten,
- Fig. 10: den Stent aus Fig. 9 in einer Seitenansicht,
- Fig. 11: einen Stent in einer wieder anderen Ausführungsform in seinem bestimmungsgemäßen Zustand, bei dem die Filamente jeweils eine alternierende Form von Bögen und Wendeln aufweisen,
- Fig. 12a: eine aus einem Material mit Thermo-Memory-Eigenschaft bestehende Muffe im Zustand vor der Verbindung zweier Filamente,
- Fig. 12b: die Muffe aus Fig. 12a in ihrem bestimmungsgemäßen, zwei Filamente umschließenden Zustand,
- Fig. 13: einen mit einer Umhüllung aus einer Faserstruktur versehenen Stent in einer Ansicht ähnlich Fig. 1,
- Fig. 14: den Stent aus Fig. 13 in einer Ansicht ähnlich Fig. 2,
- Fig. 15: verschiedene Ausgestaltungsmöglichkeiten der Überlappungsbereiche der von benachbarten Filamenten ausgehenden Fasern bei dem Stent nach Fig. 13,
- Fig. 16: ein Filament aus miteinander verdrillten Einzeldrähten mit eingedrillter Faserstruktur,
- Fig. 17: ein umhülltes Filament mit eingeschlossener Faserstruktur,
- Fig. 18: in einer ausschnittsweisen Ansicht einen Kissing-Stent im Längsschnitt,
- Fig. 19: den Kissing-Stent nach Fig. 18 mit Innenfaserung am distalen Ende in Querschnitt,
- Fig. 20: ein mit einem Klettband verbundenes Filament in einem Querschnitt,
- Fig. 21: eine ausschnittweise Ansicht eines Längsschnitts durch einen anderen Kissing-Stent,
- Fig. 22: den Kissing-Stent nach Fig. 21 im Querschnitt, und
- Fig. 23: einen Kissing-Stent in einer wieder anderen Ausführungsform,
- Fig. 24: den Kissing-Stent nach Fig. 23 im Querschnitt gemäß der Schnittlinie XXIV-XXIV in Fig. 23,
- Fig. 25: einen mit einer Umhüllung aus einem elastisch verformbaren Material versehenen Stent in einer Ansicht ähnlich Fig. 1,
- Fig. 26: die Umhüllung des Stents aus Fig. 25 in einem einem vergrößerten Ausschnitt,
- Fig. 27: einen Stent in einer anderen Ausführungsform mit voneinander in Stentlängsrichtung beabstandeten Wendelschlaufen,
- Fig. 28: einen Stent und eine Vorrichtung zur Implantation eines Stents,
- Fig. 29: einen Stent und eine Implantationsvorrichtung in einer anderen Ausführungsform in einer Seitenansicht,
- Fig. 30: einen Stent und eine Implantationsvorrichtung in einer wieder anderen Ausführungsform im Bereich des proximalen Stentendes in während der Implantation,
- Fig. 31: das distale Ende eines Stents mit einer Implantationsvorrichtung in einer wiederum anderen Ausführungsform in einer Seitenansicht und
- Fig. 32: den Stent und die Implantationsvorrichtung aus Fig. 30 in einer Draufsicht.

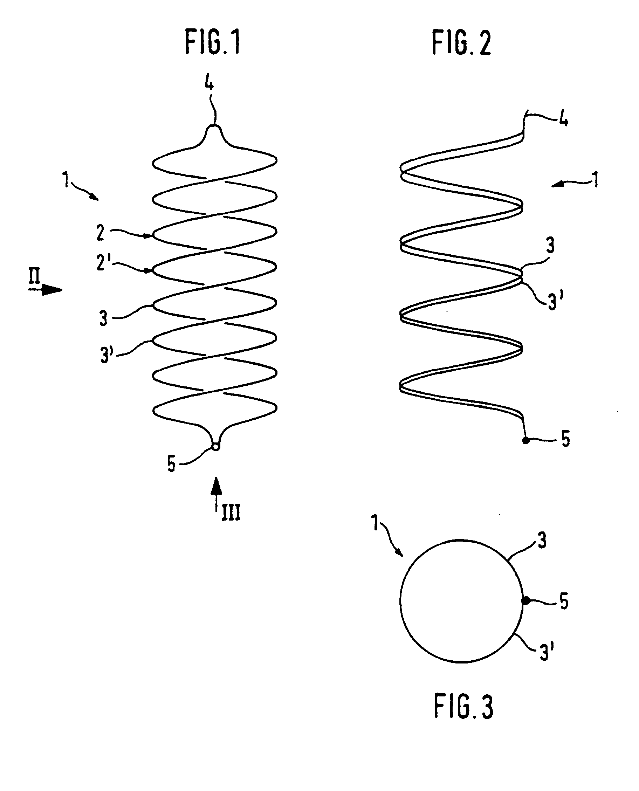

Der in den Fig. 1 bis 3 gezeigte Stent 1 besteht aus zwei Filamenten 2, 2', die zu zwei gegenläufigen Wendeln 3, 3' gewunden sind. Am distalen Ende des Stents 1 gehen die beiden Wendeln 3, 3' über eine Schlaufe 4, welche die beiden Filamente 2, 2' miteinander verbindet, ineinander über. Die Filamente 2, 2' sind somit Teile eines einzigen Drahtes. Am proximalen Ende des Stents 1 sind die beiden Filamente 2, 2' mit einem Verbindungsmittel, etwa einer die beiden Filamente 2, 2' übergreifenden Muffe 5, miteinander verbunden. Anstelle der Muffen können die Enden jedoch auch verschweißt, verlötet oder verklebt sein. Sowohl die Schlaufe 4 am distalen Ende des Stents 1 als auch die Muffe 5 am proximalen Ende sind radialaußenseitig zur Längserstreckung des Stents 1 angeordnet. Auf diese Weise wird ein über die gesamte Länge des Stents 1 gleichbleibendes Lumen offengehalten.

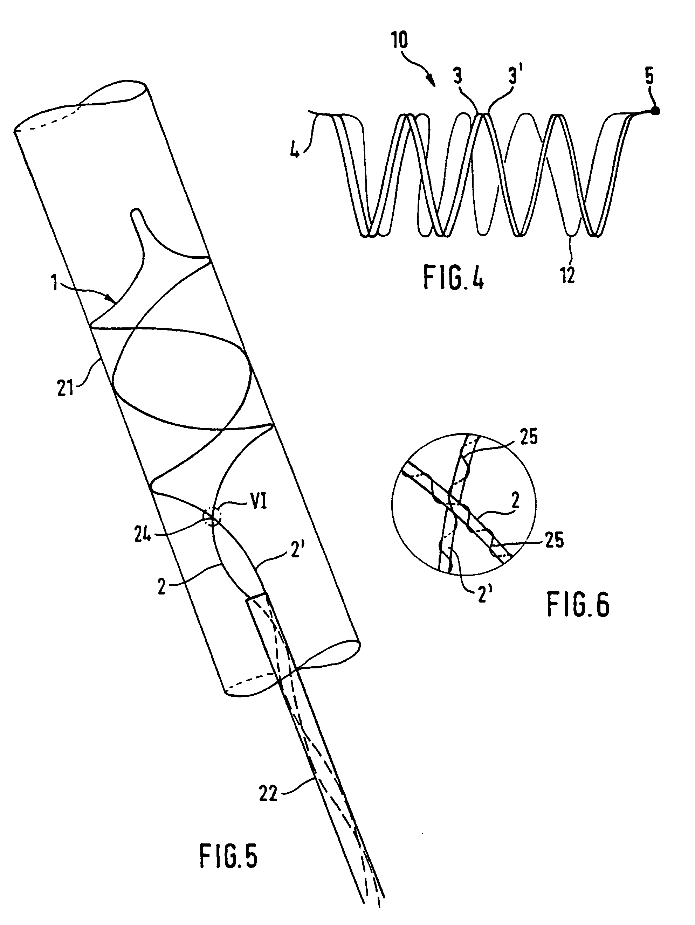

Fig. 4 zeigt einen Stent 10, der aus drei wendelförmigen Filamenten 2, 2', 12 aufgebaut ist. Die Filamente 2, 2' besitzen im wesentlichen die gleiche Steigung und bilden die aus den Fig. 1 bis 3 bekannte Doppelwendelstruktur. Das Filament 12 weist zumindest abschnittsweise eine gegenüber den Filamenten 2, 2' veränderte Steigung auf. Auf diese Weise gewährleistet das Filament 12 auch eine Gefäßabstützung im Bereich von Lücken der aus den Filamenten 2,2' bestehenden Doppelwendelstruktur.

Fig. 5 zeigt einen Stent 1 während seiner Implantation in ein Körpergefäß 21. Dabei wird der Stent 1 mit seinen Filamenten 2, 2' in unten noch näher zu beschreibender Weise mittels eines hier nicht dargestellten Pushers durch einen im Katheter 22 vorgeschoben. Die Filamente 2, 2' bestehen wahlweise aus Thermo-Memory-Draht, wie Nitinol, oder es handelt sich um langgestreckten biegsamen Draht aus hochelastischem Kunststoff. Die Filamente 2, 2' des Stents 1 treten innerhalb des zu behandelnden Körpergefäßes 21 am proximalen Ende aus dem Katheter 22 aus und springen aufgrund der genannten Thermo-Memory-Eigenschaft oder der Elastizität in die gewünschte Doppelwendelform.

Im Falle der Verwendung eines Thermo-Memory-Drahtes sollte der Katheter 22 mit einer gekühlten physiologischen Koch- salzlösung durchspült sein, um zu verhindern, daß die Filamente 2, 2' bereits innerhalb des Katheters 22 in die Wendelform springen. Andernfalls würde die das Vorschieben der Filamente 2, 2' innerhalb des Katheters 22 behindernde Reibungskraft durch die sperrigen Filament 2, 2' beträcht- lich erhöht. Hierzu kann am proximalen Ende des Katheters 22 ein Einlaßventil für die physiologische Kochsalzlösung vorgesehen und zusätzlich am distalen Ende des Katheters 22 ein Schleusenventil angeordnet sein, das zwar die physiologische Kochsalzlösung ungehindert austreten läßt, aber umgekehrt den Eintritt von warmer Körperflüssigkeit verhindert.

Zur Erhöhung der Stabilität des Stents 1 in seinem bestimmungsgemäßen Zustand sind die Filamente 2, 2' zumindest im Bereich der Kreuzungspunkte 24 mit einer Anordnung von Fäden 25 versehen. Im Ausführungsbeispiel winden sich Fäden 25 spiralförmig um beide Wendeln der Filamente 2, 2'. Im Überkreuzungsbereich 24 ist der Faden 25 an einem der Filamente 2, 2' derart von den Filamenten 2, 2' gelöst, daß zwischen dem Faden 25 und den Filamenten 2, 2' eine Beab- standung besteht, die zur Aufnahme des anderen Filaments 2, 2' ausreicht. Bei der Ausbildung der bestimmungsgemäßen Doppelwendelstruktur des Stents 1 aus der langgestreckten Implantationsstruktur verdrehen sich die die Wendeln bildenden Filamente 2, 2' zwangsläufig gegeneinander. Aus diesem Grunde muß die Verbindung der beiden Filamente 2, 2' im Überkreuzungsbereich 24 derart ausgebildet sein, daß die beiden Filamente 2, 2' gegeneinander verdrehbar sind. Mit einer Verbindung, wie sie in Fig. 6 gezeigt ist, wird eine wirksame radiale Stabilität des Stents erreicht, gleichzeitig sind die beiden Filamente 2, 2' gegeneinander verdrehbar. Zudem weisen die Filamente 2, 2' eine gewisse axiale Beweglichkeit gegeneinander auf. Dies ist insbesondere bei der Implantation des Stents in stark gekrümmte Körpergefäße von Vorteil.

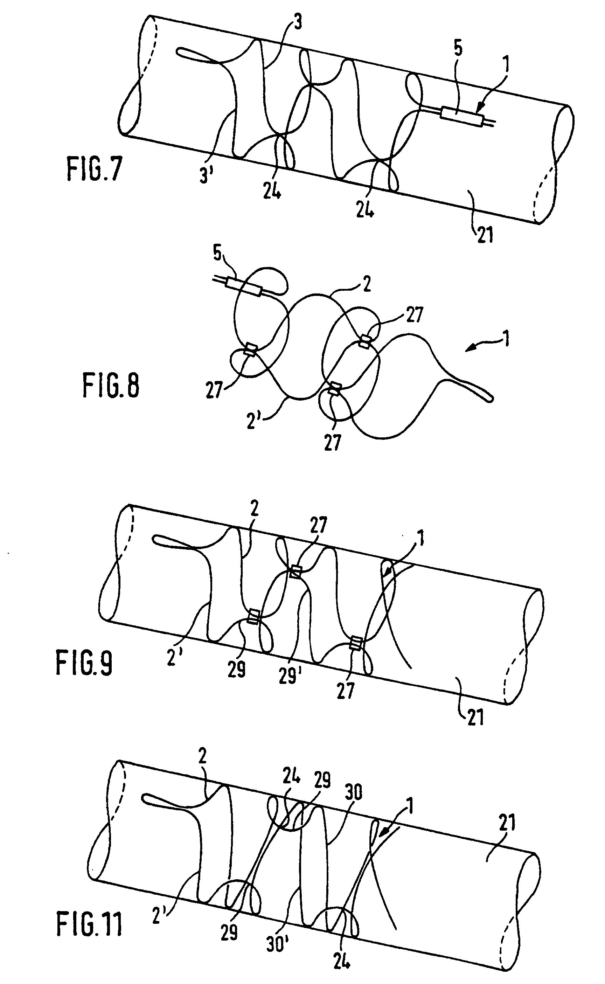

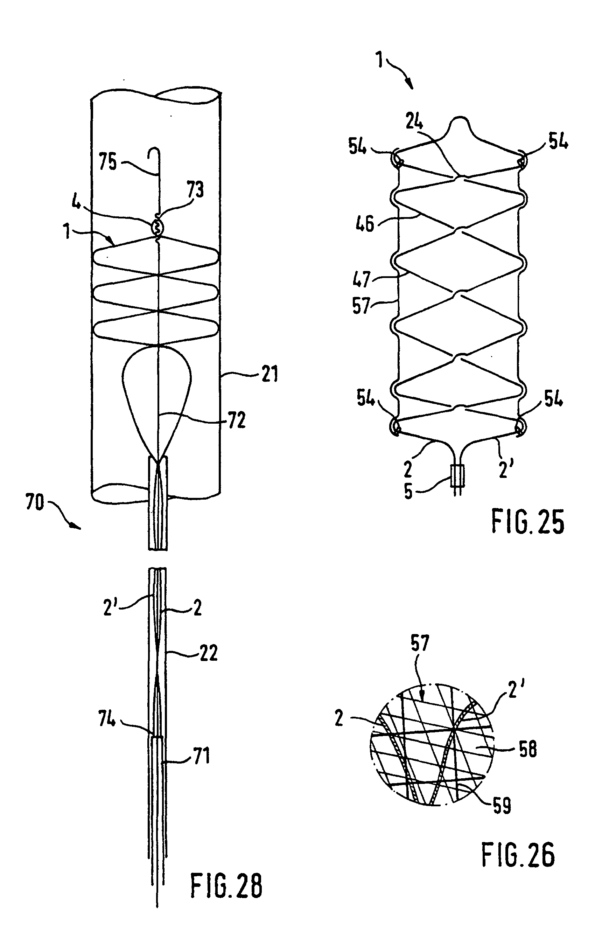

Bei der in Fig. 7 dargestellten Ausführungsform sind die einzelnen Wendelschlaufen der Wendeln 3, 3' derart umgebogen, daß sie in ihren Überkreuzungspunkten 24 etwa rechtwinklig zueinander verlaufen. Beide Wendeln 3, 3' legen sich dabei an den Innenwandungen eines Körpergefäßes 21 an. Lediglich im Bereich der Überkreuzungspunkte 24 kann eine der Wendeln 3, 3' eine Aufbiegung aufweisen, in der die andere Wendel 3, 3' aufgenommen ist, wie dies etwa in Fig. 25 gezeigt ist.

Bei der Ausführungsform nach Fig. 8 sind die Wendeln 3, 3' im Bereich ihrer Überkreuzungspunkte durch geeignete Verbindungsmittel, etwa beide Filamente 2, 2' übergreifende Muffen 27, miteinander verbunden. Als Verbindungsmittel kommen jedoch auch Fädenverbindungen in Frage, wie sie in Fig. 6 gezeigt sind.

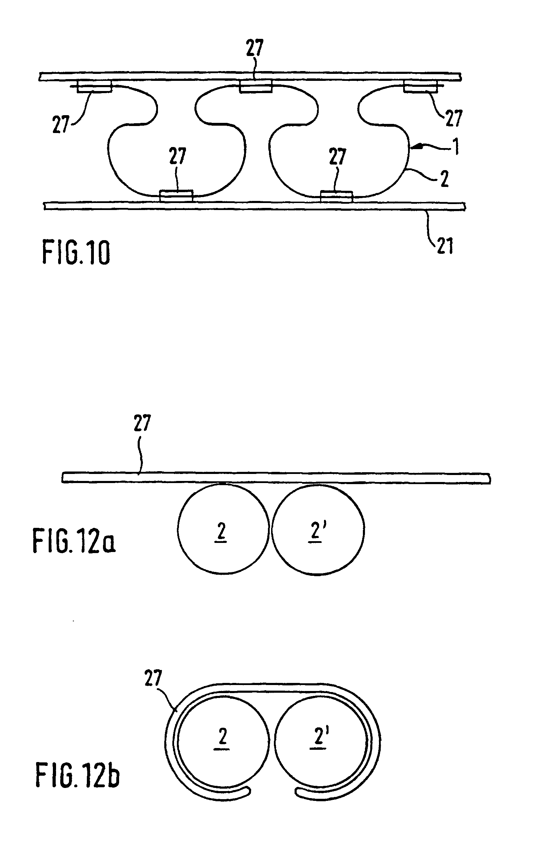

Bei der Ausführungsform nach Fig. 9 weist der distale Abschnitt des Stents 1 anstelle einer Doppelwendel eine Struktur aus gegenläufigen, radial an der Innenwandung des Körpergefäßes 21 verlaufenden Bogenabschnitten 29, 29' auf. Benachbarte Bogenabschnitte 29, 29' eines Filaments 2, 2' sind dabei in Umfangsrichtung um etwa 180° gegeneinander versetzt, wodurch eine besonders stabile Form des Stents 1 gewährleistet ist. zur weiteren Stabilisierung des Stents sind die Filamente 2, 2' in dem Bereich, wo sich die Bogenabschnitte 29, 29' maximal annähern, mittels Muffen 27 miteinander verbunden. Selbstverständlich können geeignete Muffen 27 auch im Überkreuzungsbereich 24 zweier Filamente 2, 2' eines Stents der in Fig. 7 oder Fig. 8 gezeigten Art angeordnet sein, und die Filamente können auch auf andere Art miteinander verbunden werden, etwa durch Schweißen, Kleben oder Verlöten, bzw. durch eine Kombination aus Schweißen, Löten oder Kleben mit Muffen. Je nach Anwendung sind die Drähte gegeneinander drehbar oder fest durch die genannten Verbindungsmittel miteinander verbunden.

In Fig. 10 ist gezeigt, daß - in einer Seitenansicht gesehen - die Filamente 2, 2' des Stents nach Fig. 9 jeweils zwischen zwei Punkten maximaler Annäherung der Bogenabschnitte 29, 29' - die in Fig. 10 durch die Filamente miteinander verbindenden Muffen 27 angedeutet sind - einen ungefähr "S"-förmigen Verlauf aufweisen. Im Unterschied zu der aus der WO 94/03127 vorbekannten Stentstruktur mit Filamenten, die zwischen jeweils zwei Verbindungsabschnitten parallel zueinander verlaufen, weist die Stentstruktur in dieser Ausgestaltung eine besonders hohe Flexibilität auf. Auch der Stent gemäß Fig. 7 bzw. der Stent gemäß Fig. 8 kann in dieser Ausführungsform, also mit - in Seitenansicht gesehen - zwischen jeweils zwei benachbarten Überkreuzungspunkten 24 im wesentlichen "S"-förmig verlaufenden Filamenten 2, 2' ausgebildet sein. Aufgrund des S-förmigen Verlaufs der Filamente weist der Stent eine außerordentlich hohe Flexibilität auf und insbesondere ist dadurch das Gefäß mit dem implantierten Stent komprimierbar. Eine Pulswelle kann über einen solchen Stent weitaus besser hinweglaufen als bei einem Stent mit senkrecht zur Stentlängsachse verlaufenden Filamenten.

Bei der Ausführungsform gemäß Fig. 11 weisen die Filamente 2, 2' eines Stents 1 eine alternierende Folge von Bogenabschnitten 29, 29' und Wendelschlaufen 30, 30' auf. An beiden Filamenten 2, 2' sind die Abfolgen derart versetzt, daß im Bereich der Überkreuzungspunkte 24 der beiden Filamente 2, 2' jeweils eine Wendelschlaufe 30, 30' mit einem Bogenabschnitt 29, 29' kreuzt. Auch hier

In den Fig. 12 a und b ist eine bevorzugte Ausführungsform einer jeweils zwei Filamente 2, 2' verbindenden Muffe 27 gezeigt. Die Muffe 27 besteht aus einem Material mit Thermo-Memory-Eigenschaft und ist in Fig. 12 a im Zustand vor dem Verbinden der Filamente gezeigt, also als im wesentlichen rechteckiger und eben verlaufender Zuschnitt. Nach Erreichen einer vorbestimmten Thermo-Memory-Übergangstemperatur biegt sich die Muffe 27 im wesentlichen U-förmig auf und umschließt die Filamente 2, 2', wodurch eine besonders stabile und das Verdrehen der Filamentdrähte 2, 2' gegeneinander weitgehend unterbindende Verbindung hergestellt ist. Die Muffen 27 können, angepaßt an die jeweiligen Erfordernisse, von unterschiedlicher Länge sein. So können etwa in Bereichen, in denen nur eine geringe Belastung des Stentkörpers besteht, die Zahl der Wendelschlaufen 3, 3' pro Längeneinheit des Stents in einfacher Weise dadurch vermindert werden, daß im Überkreuzungsbereich eine entsprechend langgestreckte Muffe zur Verbindung der beiden Filamente 2, 2' eingesetzt wird. Kurze Muffen sind dagegen im Bereich der Stentenden angeraten, da dort die Stentbelastung besonders hoch ist und eine hohe Wendeldichte wünschenswert.

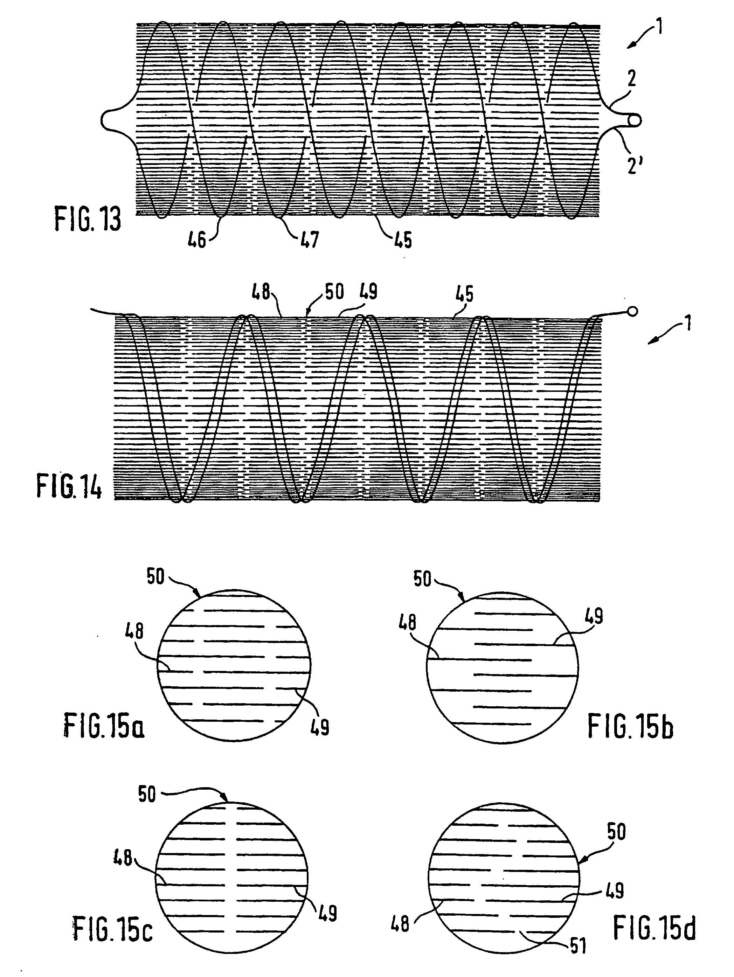

Bei der in den Fig. 13 und 14 gezeigten Ausführungsform sind die Filamente 2, 2' des Stents 1 mit einer Gewebeoder Metallstruktur 45 derart verbunden, daß sich eine tubusförmige Ummantelung des Stents 1 ergibt. Die von der Gewebe- oder Metallstruktur 45 gebildete Ummantelung ist aufgrund ihrer Porösität diffusionsfähig, hingegen bei der Verwendung textiler Gewebeteile ist die Ummantelung sehr feinporig, hingegen bei der Verwendung von Metallgeweben sehr großporig ausgebildet. Die Entscheidung, eine Gewebe- oder eine Metallstruktur zu verwenden, ist abhängig von der ge- wünschten Steifigkeit des Stents 1 und den benötigten Diffusionseigenschaften.

Die von benachbarten Wendelschlaufen 46, 47 ausgehenden Fasern bzw. Gewebeteile grenzen in einem Überlappungsbereich 50 zwischen den benachbarten Wendelschlaufen 46, 47 aneinander. Die Art und Weise, wie die einzelnen Gewebeteile und/oder Fasern aneinander angrenzen, kann verschiedenartig ausgebildet sein, wie Fig. 15 zeigt.

In Fig. 15 ist ausschnittsweise der Überlappungsbereich 50 zwischen von benachbarten Wendelschlaufen 46, 47 ausgehenden Fasern bzw. Gewebeteilen der Gewebestruktur 45 in verschiedenen Ausführungsformen a) - d) am Beispiel aneinander angrenzender Fasern 48, 49 gezeigt. Die dichteste und festeste Verbindung der Gewebeteile bzw. Fasern miteinander besteht in einer Überlappung der von benachbarten Wendelschlaufen 46, 47 ausgehenden Fasern 48, 49. Dies zeigen die Fig. 15 a und 15 b. Während bei der Ausführungsform nach Fig. 15 a die Faserdichte der Gewebestruktur 45 über den Stent hinweg konstant bleibt, ergeben sich bei der Ausührungsform nach Fig. 15 b Zonen größerer Dichte und damit größerer Dicke der Gewebestruktur 45 im Bereich der Überlappung 50 der Fasern 48 und 49.

Eine andere Ausführungsform zeigt Fig. 15 c, bei der sich die Fasern nicht überlappen, sondern lediglich an ihren Enden berühren, wodurch eine über den Außenumfang des Stents 1 kreisförmige Übergangszone entsteht. Ein so ausgebildeter Stent besitzt eine geringere Steifigkeit und Festigkeit gegenüber den Ausführungsformen nach 15 a und 15 b, erfordert jedoch einen erheblich geringeren Materialaufwand und ist damit gegenüber den vorgenannten Ausführungsformen deutlich leichter. Zudem kann der Einführkatheter ein kleineres Lumen aufweisen. Bei der Ausführungsform nach Fig. 15 d schließlich liegt ebenfalls keine Überlappung der Fasern 48, 49 vor, sondern die Fasern 48, 49 sind derart ausgebildet, daß ihre Enden im Übergangsbereich 50 eine wellenförmige Übergangslinie 51 bilden.

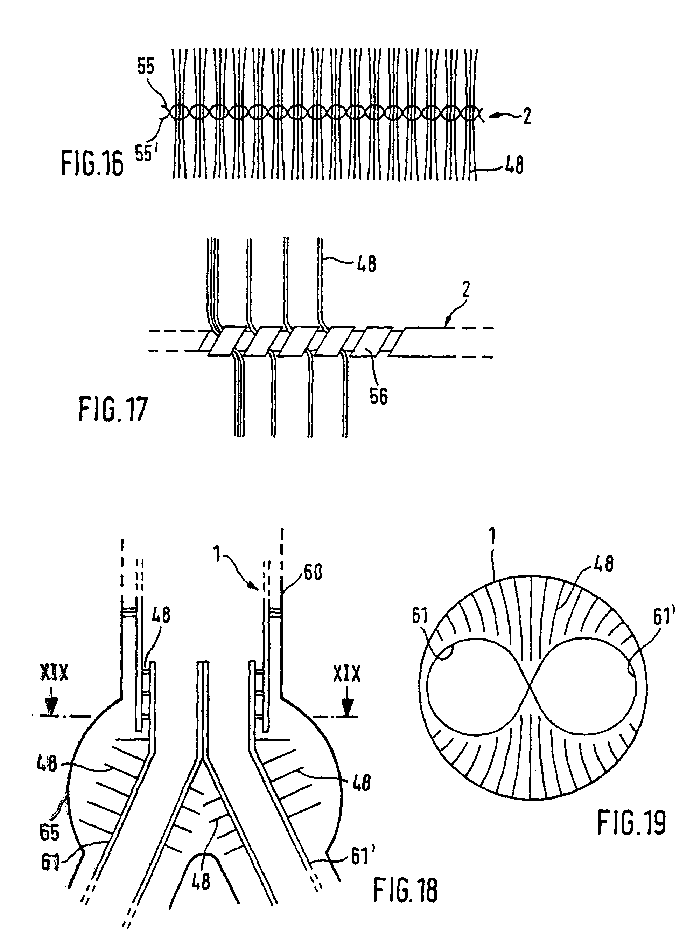

Die Fig. 16 und 17 zeigen verschiedene Möglichkeiten, eine Gewebestruktur mit den Filamenten 2, 2' zu verbinden. Nach Fig. 16 ist das Filament 2 mit sich radial vom Filament 2 forterstreckenden Fasern 48 versehen. Die Fasern 48 bestehen zumeist aus Dacron- oder Teflon-Material und können abschnittsweise oder je nach Vorzugsrichtung unterschiedliche Länge haben. In Fig. 16 ist das Filament 2 aus miteinander verdrillten Teilfilamenten 55, 55' hergestellt. Die Teilfilamente 55, 55' bestehen dabei ebenfalls aus Thermo-Memory-Draht oder hochelastischen Kunststoffen. Zwischen den einzelnen Teil- filamenten 55, 55' sind mittels der Verdrillung dieser Teilfilamente 55. 55' die Dacron-Fasern 48 gehalten. Bei dieser Ausführungsform werden keine körperfremden Klebstoffe mit zweifelhafter oder begrenzter Haltbarkeit benötigt.

Bei der in Fig. 17 dargestellten Ausführungsform ist ein Filament 2 von einer Umhüllung 56 umwickelt. Diese Umhüllung 56 kann ebenfalls aus einer textilen oder metallenen Gewebestruktur bestehen. Es kann sich dabei auch um einen Hilfsfaden oder Hilfsdraht handeln und etwa in der in Fig. 6 gezeigten Art mittels eines Fadens 25 am Filament 2 befestigt sein. Auch bei dieser Ausführungform ist die Umhüllung 56 derart um das Filament 2 geschlungen, daß sich radial vom Filament forterstreckende Fasern 48 eingeschlossen sind.

Die sich bei den Ausführungsbeispielen nach den Fig. 16 und 17 von einem Filament 2 forterstreckenden Fasern 48 dienen ebenfalls der Ausbildung einer Umhüllung zwischen den einzelnen Wendelschlaufen 30, 30' des Stents 1. Das von den einzelnen Fasern 48 gebildete Geflecht kann aber auch gezielt zur Erzeugung von Thrombosen beispielsweise innerhalb einer krankhaften Gefäßaussackung, etwa einem Aneurysma, eingesetzt werden. Gemäß einer anderen Ausführung können die Fasern 48 auch gezielt in das Innere des Stents 1 hineinragen, um einen gewünschten Gefäßverschluß zu beschleunigen.

Die Fig. 18 und 19 zeigen einen Stent für eine spezielle Behandlungsmethode bei Gefäßerkrankungen im Bereich von Gefäßaufzweigungen. Dafür wird die sogenannte Kissing-Stentmethode erfolgreich eingesetzt. Hierbei wird ein Hauptstent 1 mit größerem Durchmesser in das sich aufzweigende Gefäß 60 implantiert und anschließend distalseitig durch die abzweigenden Gefäße je ein weiterer Hilfsstent 61, 61' distalseitig in den zunächst implantierten Stent 1 eingeschoben. Im Falle einer pathologischen Aorta abdominalis mit einem Aneurysma wird je ein Hilfsstent 61, 61' durch die Arteriae femorales in das distale Lumen des bereits implantierten Hauptstents 1 eingeschoben. Bislang war dies problematisch, da sich im Bereich der Abzweigung der beiden distalseitig eingeschobenen Hilfsstents 61, 61' eine Leckage ergab, weil die nachträglich distalseitig eingeschobenen Hilfsstents 61, 61' nicht das gesamte Lumen des ursprünglich implantierten Hauptstents 1 ausfüllten. Im Falle der abschnittsweisen Anordnung von nach innen ragenden Fasern 48 gemäß Fig. 19 am distalen Ende des Hauptstents 1 kann die beschriebene Leckage wirksam dadurch beseitigt werden, daß es zu einer beschleunigten Thrombosierung innerhalb der Leckagezonen kommt. Die Fasern 48 können jedoch auch an den Hilfsstents 61, 61' angeordnet sein und radial nach außen weisen. Je nach Dichtigkeit und radialer Erstreckung der Fasern 48 ist es in diesem Falle unter Umständen möglich, gänzlich auf den Hauptsent 1 zu verzichten. Eine wirksame Thrombosierung erfolgt dann unmittelbar zwischen der Wand des Körpergefäßes 60 und den Hilfsstents 61, 61'. Selbstverständlich kann dabei anstelle der Fasern 48 auch ein anderer Dichtungsbelag, etwa eine Gewebestruktur, eingesetzt werden. Die beiden Hilfsstents 61, 61' können dabei auch aus einem aus einer Doppelwendelstruktur gebildeten Stent 35 bestehen und in dem Bereich, in den sie in den Hauptstent eingeschoben sind, den Querschnitt einer "Acht" aufweisen. Dies zeigt Fig. 19.

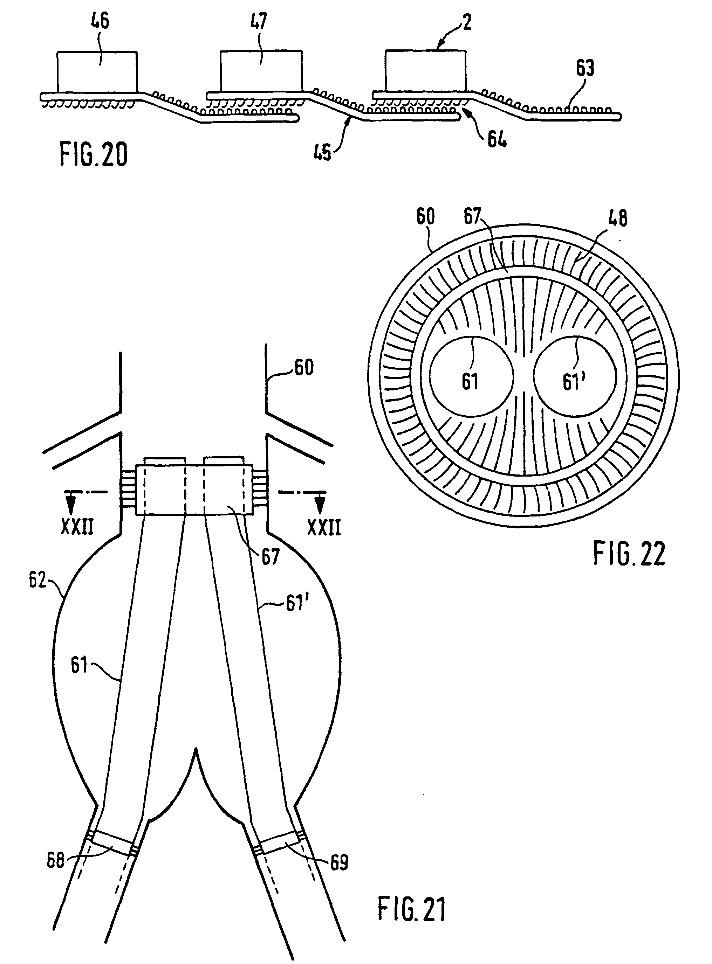

Gemäß Fig. 20 kann die mit dem Filament 2 verbundene Gewebe- oder Metallstruktur 45 mit einem Klettband 63 verbunden sein. Im Bereich der Überlappungen der von einander benachbarten Wendeln ausgehenden Gewebe- oder Metallstrukturen 45 kommt es zur Ausbildung von Klettverschlüssen 64. Hierdurch wird eine verbesserte Quersteifigkeit des Stents 1 erreicht. Darüber hinaus kann sich die Gewebestruktur 45 mit zunehmendem Abstand von dem Filament 2 nach außen hin verjüngen, um im Gegensatz zu der nicht maßstäblichen Darstellung in Fig. 20 eine gleichmäßige Außenkontur des Stents 1 zu gewährleisten. Der in Fig. 20 gezeigte rechteckige Querschnitt der Stent-Filamente ermöglicht dabei eine besonders feste Verbindung des Klettverschlusses mit dem Filament. Generell gewährleistet ein rechteckiger Filament-Querschnitt eine stabilere Befestigung von Hüllen und/oder Fasermaterial an die Stent-Filamente. Alternativ zu dem in Fig. 20 gezeigten rechteckigen Filament-Querschnitt können jedoch auch Filamente mit einem "D"-förmigen Querschnitt eingesetzt werden. Um eine besonders hohe Dichtigkeit der Klettverschlüsse 64 zu erzielen, sollte nach erfolgter Implantation in den Stent ein Ballonkatheter eingeführt werden, der im Bereich der Klettverschlüsse 64 kurzzeitig in seinen Aufweitungszustand gebracht wird, wodurch die Klettbänder 63 gegeneinander gepreßt werden.

Ergänzend zu den Fig. 18 und 19 ist in den Fig. 21 und 22 eine weitere Ausgestaltung der Erfindung zum Durchführen der Kissing-Stent-Methode dargestellt. Fig. 21 zeigt einen Längsschnitt durch ein infrarenales Aortenaneurysma 65, das bis in die Bifurkation reicht. Auch bei dieser Ausgestaltung sind zwei Nebenstents 61,61' im Bereich der Gefäßaufzweigung vorgesehen, die die Gefäßaufzweigung mitvollziehen. Anstelle des in Fig. 18 gezeigten Hauptstents 1 sind bei der Ausführung gemäß den Fig. 21 und 22 lediglich Dichtungsstents 67, 68, 69 vorgesehen, die nur eine geringe Längenerstreckung haben. Der in Fig. 22 dargestellte Querschnitt im Bereich des Aneurysmahalses zeigt den Dichtungsstent 67 in einer gegenüber Fig. 21 vergröβerten Ansicht. Die sich von dem Dichtungsstent 67 forterstreckenden Fasern 48 ragen einerseits radial nach innen in das Gefäßlumen hinein und sind mit den Nebenstents 61, 61' verbunden und andererseits ragen sie radialaußenseitig über den Außenumfang des Dichtungsstents 67 hinaus und schmiegen sich an der Wand des Körpergefäßes 60 im Bereich vor dem Aneurysma an. Hierdurch wird sowohl eine Leckage zwischen den beiden Hilfsstents 61, 61' untereinander als auch zu der Aortenwand im Bereich des Aneurysmas 65 vermieden. Die Funktion der Dichtungsstents 68, 69 ist analog zur Funktion des Dichtungsstents 67 zu verstehen. In vorteilhafter Ausgestaltung können die eingebrachten Nebenstents 61, 61' auch einen ovalen oder D-förmigen Querschnitt aufweisen, um eine bessere Ausfüllung des Aorten-Lumens, und somit eine bessere Dichtwirkung, zu erzielen. Auf diese Weise wird die Gefahr einer Aortenruptur am Aneurysma wirksam unterbunden.

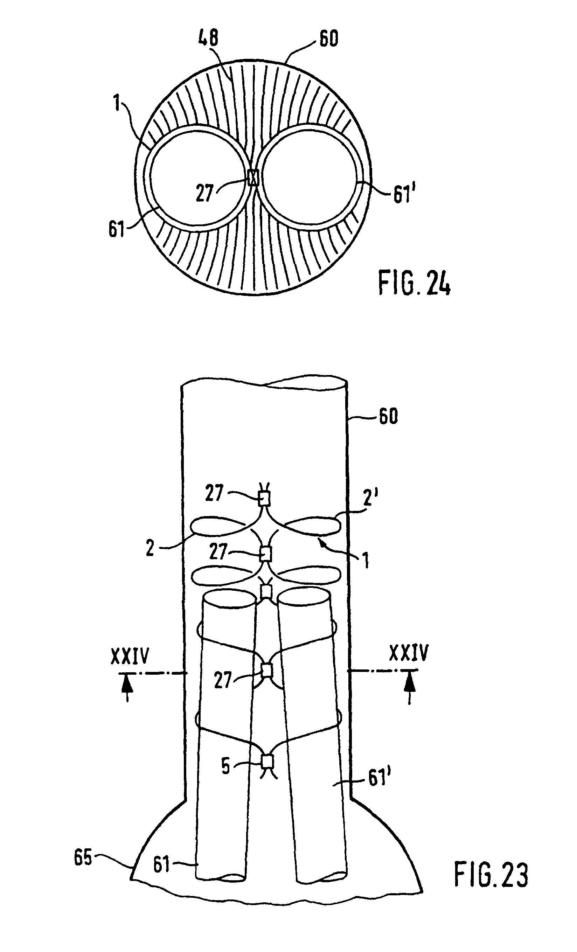

Eine wiederum andere Möglichkeit, mit Hilfe von Doppelwendelstents einen Kissing-Stent zu verwirklichen, zeigen die Fig. 23 und 24. In Fig. 23 ist der Kissing-Stent wiederum in seinem Implantationszustand in einem Körpergefäß 60 im Bereich eines Aneurysmas 62 veranschaulicht. Bei diesem Kissing-Stent weist der Hauptstent 1 in seinem distalen Abschnitt ein Lumen, in seinem proximalen Abschnitt dagegen zwei Lumina auf, in die während der Implantation die in Fig. 23 nur abschnittsweise angedeuteten Nebenstents 61, 61' eingeschoben werden. Die beiden Filamente des Hauptstents 1 sind in diesem proximalen Abschnitt derart ausgebildet, daß der Stent 1 im Querschnitt - wie in Fig. 24 gezeigt - die Form einer "Acht" aufweist. Auf diese Weise wird eine sehr zuverlässige und stabile Verbindung zwischen Hauptstent 1 und Nebenstents 61, 61' geschaffen. Alternativ zu der "Acht"-förmigen Struktur kann der Stent 1 in seinem proximalen Abschnitt auch in Form zweier gegenläufiger, nebeneinander liegender und sich punktweise zumindest annähernd berührender Einzelwendeln vorliegen. Um eine der Ausführungsform nach Fig. 23 entsprechende Stabilität zu erreichen, müssen in diesem Falle allerdings die gegenläufigen Wendeln an ihren Berührungspunkten miteinander etwa mittels Muffen 27 oder der in Fig. 6 gezeigten Fäden 25 verbunden sein.

Wie in Fig. 24 gezeigt, besteht auch bei derartigen Kissing-Stents die Möglichkeit, mittels radial von den Filamenten des Hauptstents 1 und/oder der Nebenstents 61, 61' in Richtung auf die Wandung des Körpergefäßes 60 vorstehender Fasern 48 und/oder Gewebestrukturen eine beschleunigte Thrombosierung zu erreichen. Um das Lumen des Körpergefäßes 60 besser auszunutzen, können die beiden Lumina des proximalen Abschnitts des Stents 1 auch jeweils einen ovalen Querschnitt, anstelle des in Fig. 24 gezeigten kreisförmigen Querschnitts, aufweisen.

In Fig. 25 weist der Stent 1 anstelle einer mit den Filamenten 2, 2' verbundenen Faser- oder Gewebestruktur eine membranartige Umhüllung 57 auf, die den Stent 1 radialaußenseitig umschließt und die mittels Nähten 54 mit den Filamenten 2, 2' verbunden ist. Die Nähte 54 sollten vorzugsweise an den Enden des Stents 1 befestigt sein, sie können jedoch auch an anderen Bereichen des Stentkörpers angeordnet sein.

Die mit dem proximalen Ende des Stents 1 mittels einer Muffe 5 verbundenen Filamente 2, 2' bilden gegenläufige Wendeln, deren einzelne Wendelschlaufen 46, 47 in Fig. 25 über die Längserstreckung des Stents 1 hinweg unterschiedliche Steigungen aufweisen. Die Umhüllung 57 besteht aus einem textilen Gestricke, kann jedoch auch aus körperverträglichem, hochelastischem Material, vorzugsweise Kunststoff, Latex oder Silikon gebildet sein. Daher schmiegt sich die Umhüllung 57 einerseits eng an die Wendelschlaufen 46, 47 der Filamente 2, 2' an, so daß der Stent in seinem bestimmungsgemäßen Zustand nahezu radialfest im Körpergefäß implantiert ist, zum anderen weist die Umhüllung 57 im Bereich zwischen benachbarten Wendelschlaufen 46, 47 ein Lumen auf, das zumindest annähernd dem Lumen der Wendelschlaufen 46, 47 entspricht. Die Umhüllung 57 umschließt auch, ohne eine Beeinträchtigung der Stabilität, die im Bereich der Überkreuzungen 24 im Ausführungsbeispiel nach Fig. 25 gezeigte Aufbiegung eines der beiden Filamentdrähte 2, 2'.

Fig. 26 zeigt in einem vergrößerten Ausschnitt die Umhüllung 57 des Stents 1 aus Fig. 25 in dessen bestimmungsgemäßem Implantationszustand. in dies.em Zustand verlaufen die Maschen 58 rautenförmig über den Außenumfang des von den Filamenten 2 gebildeten Stentkörpers. Je nach Aufweitungsgrad des Stents in seiner Implantationslage kann der von den Seiten der rautenförmigen Maschen 58 eingeschlossene Winkel unterschiedlich ausgebildet sein, bis hin zu einer annähernden Rechteckform der Maschen 58. Im gestreckten Zustand des Stents dagegen verlaufen die die Maschen 58 bildenden Fäden der Umhüllung 57 annähernd parallel zueinander. Um im Implantationszustand ein Eindringen der Textilfäden der Umhüllung 57 in den Zwischenbereich zwischen den Wendelschlaufen 3 der Filamente zu verhindern, ist die Umhüllung 57 zusätzlich mit, vorzugsweise aus Metall bestehenden, Verstärkungsfäden 59 versehen, die im Ausführungsbeispiel nach Fig. 26 ebenfalls maschenartig in der Umhüllung 57 eingewirkt sind und im Implantationszustand des Stents 1 ungefähr rechtwinklig zueinander verlaufen.

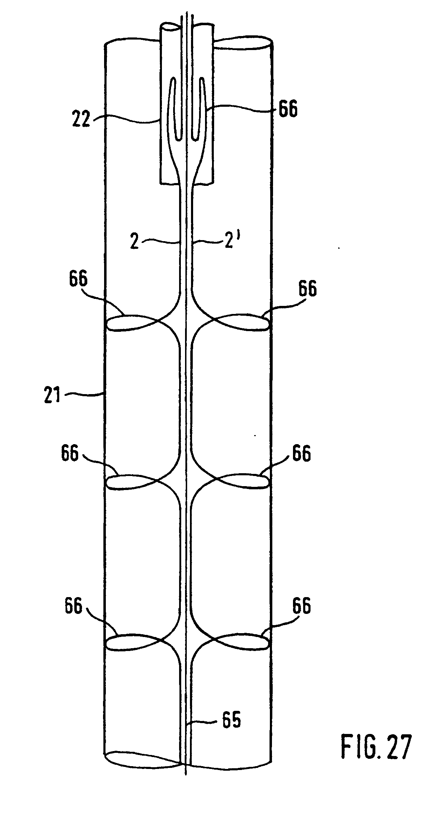

Fig. 27 zeigt einen erfindungsgemäßen Stent 1 in einer wieder anderen Ausführungsform, bei dem die Filamente 2, 2' über den Großteil der Längserstreckung des Stents 1 parallel zueinander angeordnet sind, jedoch in vorbestimmten Abständen sich zu zueinander gegenläufigen Wendelschlaufen 66, 66' aufweiten. Bei der Implantation des Stents 1 mittels eines Katheters 22 sind die Wendelschlaufen 66, 66' umgebogen und liegen eng an den parallelen Abschnitten der Filamente 2, 2' an. Somit sind die axialen Beabstandungen der Wendelschlaufen 66, 66' am Stent 1 derart bemessen, daß im gestreckten Zustand des Stents einander axial benachbarte Wendelschlaufen 66, 66' nicht berühren, um den zur Implantation erforderlichen Innendurchmesser des Katheters 22 so gering wie möglich zu halten. Ähnlich wie der in Fig. 25 gezeigte Stent ist auch der in Fig. 27 gezeigte Stent mit einer Umhüllung 57, die vorzugsweise aus einem Textilgestricke beteht, umgeben. Alternativ zu der in Fig. 27 gezeigten Ausführungsform können die gegenläufigen Wendelschlaufen 66 auch an einer Seite des Hauptdrahtes angeordnet sein.

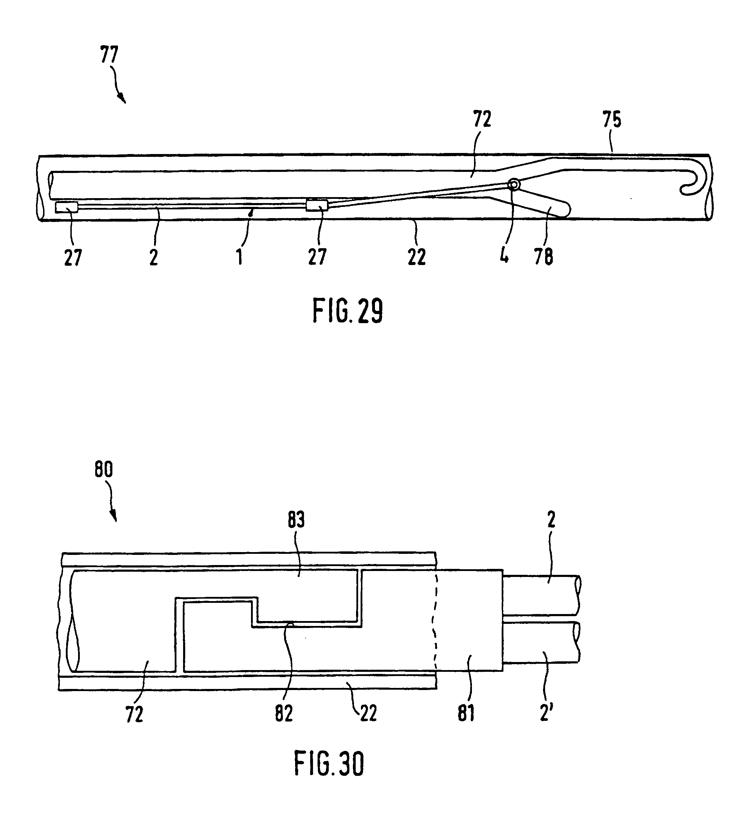

Fig. 28 schließlich zeigt eine vorteilhafte Ausgestaltung einer Implantationsvorrichtung 70. Der Stent 1 wird dabei über einen Katheter 22 als langgestrecktes Doppelfilament in das Körpergefäß 21 eingebracht, wo er seine bestimmungsgemäße Doppelwendelstruktur einnimmt. Zwei Pusher 71, 72 werden verwendet, um den Stent 1 in seine vorbestimmte Position zu bringen. Der äußere Pusher 71 dient dabei dazu, die sich an dessen vorderem Ende 74 abstützenden Stentfilamente 2, 2' durch den Katheter 22 hindurch zu einem bestimmungsgemäßen Ort im Körpergefäß 21 zu schieben. Durch den äußeren Pusher 71 erstreckt sich ein konzentrisches Lumen hindurch, durch die der innere Pusher 72 geführt ist. Der innere Pusher 72 besteht aus einem dünnen, jedoch festen Draht, der an seinem distalen Ende einen Gewindeabschnitt 73 aufweist und mit letzterem mit dem distalen Ende des Stents 1 verbunden ist. Am distalen Ende des Gewindeababschnitts 73 des inneren Pushers 72 ist ein flexibler Führungsdraht 75 angeordnet, dessen Spitze umgebogen ist, um beim Einführen des inneren Pushers in das Gefäß Gefäßverletzungen zu vermeiden.

Beim Einführen des Stents 1 in das Gefäß 21 werden zunächst beide Pusher 71, 72 bei gestrecktem Stent 1 im Katheter 22 vorgeschoben. Sobald ein erster Abschnitt des Stents 1 im Körpergefäß seine bestimmungsgemäße Doppelwendelstruktur eingenommen hat, wird der Stent durch den inneren Pusher 72 koaxial zur Wandung des Gefäßes 21 gehalten. Ein Zurückspringen oder ein Vorspringen des Stents 1 im Körpergefäß 21 wird dadurch vermieden. Der Gewindeabschnitt 73 des inneren Pushers 72 ist an der die beiden Filamente 2, 2' des Stents 1 miteinander verbindenden Endschlaufe 4 fest aber lösbar mit dem Stent 1 verbunden. Während der Katheter 22 zurückgezogen und gleichzeitig der Stent mittels des äuβeren Pushers 71 vorgeschoben wird, wird das distale Stentende durch den inneren Pusher 72 gehalten. Wenn der Stent 1 vollständig ins Körpergefäß 21 eingebracht worden ist, wird der innere Pusher 72 von der Endschlaufe 4 des Stents 1 gelöst. Selbstverständlich könnte auch ein Gewinde an der Stentspitze angebracht sein, um eine zuverlässigere Befestigung des inneren Pushers 72 am Stent 1 zu erhalten.

Es ist auch denkbar, daß der äußere Pusher 71 an seinem vorderen Ende 74 mit dem Stent 1 lösbar, z.B. mittels einer Gewindeverschraubung, verbunden ist. Eine derartige Ausbildung ermöglicht es, einen implantierten Stent nach einer gewissen Implantationsdauer wieder aus einem Körpergefäß 21 zu entfernen. Eine weitere alternative Ausführungsform der Implantationsvorrichtung 70 sieht vor, anstelle der koaxial zueinander angeordneten Pusher 71, 72 diese nebeneinander im Katheter vorzusehen, wobei ein dem inneren Pusher 72 entsprechender erster Pusher den anderen an dessen distalen Ende um eine vorbestimmmte Länge überragt.

Die in Fig. 29 veranschaulichte Implantationsvorrichtung 77 weist einen Katheter 22 und einen darin axialbeweglich aufgenommenen Pusher 72 auf, der, ebenso wie der Pusher 72 der Implantationsvorrichtung aus Fig. 26, mit einem an seinem distalen Ende aufgebogenen Führungsdraht 75 versehen ist. An dem sich an den Führungsdraht 75 anschließenden vorderen Abschnitt des Pushers 72 ist eine gabelförmige Erweiterung 78 angeordnet, die zum Umgreifen der die Filamente 2, 2' des zu implantierenden Stents 1 an dessen distalem Ende abschließenden Schlaufe bzw. die Filamente 2, 2' miteinander verbindenen Muffen 27 vorgesehen ist.

Nach lagerichtiger Plazierung des Katheters 22 im - in Fig. 29 nicht dargestellten - Körpergefäß 21 wird der Stent 1 an einer die Filamente 2, 2' am distalen Stentende abschließenden Schlaufe 4 mittels des Pushers 72 in das Körpergefäß vorgeschoben. Nach lagerichtiger Plazierung des distalen Stentendes wird der Pusher 72 in den Katheter 22 bis hinter die nachfolgende, die Filamente 2, 2' verbindende Muffe 27 zurückgeführt. Sodann wird der Pusher 72 erneut vorgeschoben, wobei er mit seiner gabelförmigen Erweiterung 78 die nachfolgende Muffe 27 übergreift, mit der Folge, daß die im Katheter befindlichen Teile des Stents an dieser Muffe 27 mittels des Pushers 72 aus dem Katheter 22 herausgeschoben werden können. Durch die sukzessive Betätigung des Pushers 72 an den die Filamente 2, 2' des Stents 1 verbindenden Muffen 27 ist eine sehr zuverlässige lagerichtige Plazierung des Stents 1 im Körpergefäß möglich.

Während die in Fig. 29 gezeigte Implantationsvorrichtung 77 auf das distale Ende des Stents einwirkt, ist mit der in Fig. 30 ausschnittsweise veranschaulichten Implantationsvorrichtung 80 eine Führung eines Stents 1 an dessen proximalem Ende möglich.

Auch die Implantationsvorrichtung 80 weist einen Katheter 22 und einen darin axialbeweglich aufgenommenen Pusher 72 auf. Am distalen Pusherende ist ein Hakenelement 83 angeordnet. Gleichzeitig sind die Filamente 2, 2' des zu implantierenden Stents 1 an dessen proximalem Ende mittels einer Endmuffe 81 miteinander verbunden, die wiederum mit einer quer zur Axialerstreckung des Stents verlaufenden Kerbe 82 versehen ist, die formschlüssig mit dem Hakenelement 83 des Pushers 72 verbindbar ist.

Während der Implantation wird das distale Stentende mittels des Pushers 72 aus dem Katheter 22 herausgeschoben.

Bis zur endgültigen lagerichtigen Plazierung des Stents 1 verbleibt dessen proximales Ende jedoch im Katheter 22 und ist somit axialfest mit dem Pusher 72 an dessen Hakenelement 83 verbunden. In dieser Situation ist jederzeit das vollständige Zurückziehen des Stents 1 in den Katheter 22 möglich.

Das Ablösen des Stents 1 vom Pusher 72 erfolgt in einfacher Weise dadurch, daß nach der lagerichtigen Plazierung des Stents 1 im Körpergefäß 21 auch das proximale Ende des Stents 1 aus dem Katheter 22 hinausgeschoben wird. Der Stent 1 und der Pusher 72 sind dann senkrecht zur Axialerstreckung des Katheters 22 frei gegeneinander beweglich und die Endmuffe 81 ist somit vom Hakenelement 83 ablösbar.

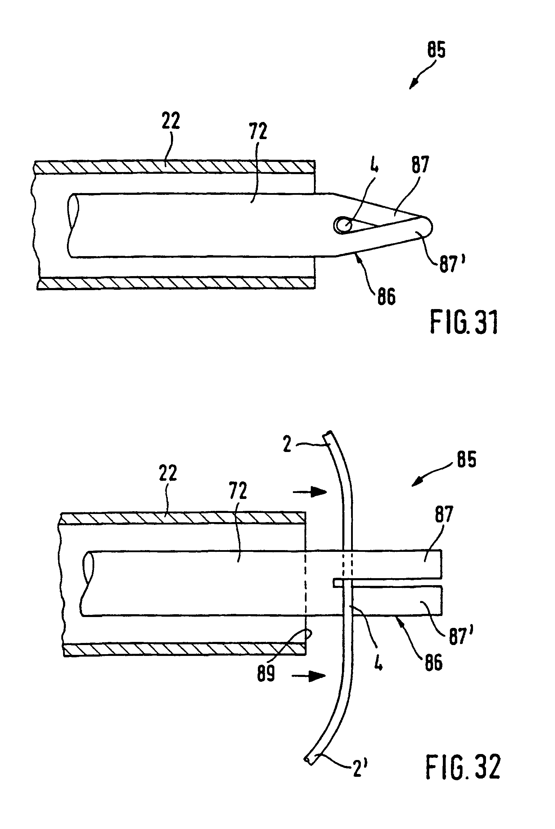

Eine wieder andere Implantationsvorrichtung 85, die zur exakten Plazierung des distalen Stentendes in einem Körpergefäß vorgesehen ist, ist in den Fig. 31 und 32 gezeigt. Die Implantationsvorrichtung 85 ist wiederum mit einem Katheter 22 und einem axialbeweglich darin aufgenommenen Pusher 72 versehen. Der Pusher weist einen zangenförmigen Vorderabschnitt 86 auf, dessen Zangenelemente 87, 87' das distale Ende eines zu implantierenden Stents 1, etwa an einer den Stent 1 distalseitig abschließenden Schlaufe 4 oder an einer die Filamente 2, 2' des Stents 1 an dessen distalem Ende verbindenden Muffe 5, 27 übergreifen. Im Gegensatz zu der in Fig. 29 gezeigten Implantationsvorrichtung 77 ist mit der in Fig. 31 gezeigten Implantationsvorrichtung 85 somit eine Vorwärts- und Rückwärtsbewegung des Stents 1 mit Hilfe des Pushers 72 möglich, wodurch eine etwaige Fehlpositionierung des distalen Endes des Stents 1 im Körpergefäß 21 korrigiert werden kann.

Das Ablösen des Stents 1 vom Pusher 72 erfolgt dadurch, daß zunächst der Katheter 22 soweit im Körpergefäß 21 zurückgezogen wird, daß sich zumindest ein Teil der Wendelschlaufen des Stents 1 zu ihrer bestimmungsgemäßen Implantationslage entfalten, wie dies in Fig. 32 angedeutet ist.

Durch abermaliges Vorschieben des Katheters 22 beaufschlagt dieser den Stent 1 in axialer Richtung mit einer - in Fig. 32 durch Pfeile dargestellten - Kraft, dievermittelt über die Filamente 2, 2' am distalen Ende dazu führt, daß die Zangenelemente 87, 87' auseinandergedrückt werden und somit die Schlaufe 4 freigeben.

## Patentansprüche

1. Stent zur Behandlung pathologischer Körpergefäße, der dauerhaft implantierbar ist und und erst am Implantationsort während der Implantation seine bestimmungsgemäße Form einnimmt, und mehrere Filamente umfasst, wobei die Filamente (2, 2') im bestimmungsgemäßen Zustand des Stents (1) an wenigstens einer im Bereich des Außenumfangs des Stents (1) liegenden Verbindungsstelle (4, 5) fest miteinander verbunden sind, wobei wenigstens zwei der Filamente (2, 2') in ihrem bestimmungsgemäßen Zustand über wenigstens einen Teil der Längserstreckung des Stents (1) in der Form zueinander gegenläufiger Wendeln (3, 3') vorliegen und diese Filamente (2, 2') der zueinander gegenläufigen Wendeln (3, 3') aus einem einzigen Filamentdraht bestehen, der an einer ersten der wenigstens einen Verbindungsstelle der beiden Wendeln (3, 3') einen Knick oder eine Schlaufe (4) zur Ausbildung einer gegenläufigen wendel (3, 3') aufweist, **dadurch gekennzeichnet, dass**
der Stent (1) in Form wenigstens zweier langgestreckter Filamente (2, 2') mittels einer Implantationsvorrichtung (70) in ein Körpergefäß (21, 60) einführbar ist und die erste der wenigstens einen Verbindungsstelle der beiden Wendel jeweils an einem der stirnseitigen Enden des Stents (1) angeordnet ist.

2. Stent nach Anspruch 1, **dadurch gekennzeichnet, dass** die Filamente (2, 2') aus einem Material mit superelastischen Eigenschaften und/oder Thermo-Memory -Eigenschaft bestehen.

3. Stent nach Anspruch 1 oder 2, **dadurch gekennzeichnet, daß** die Filamente (2, 2') der gegenläufigen wendeln (3, 3') einstückig aus einem rohrförmigen Werkstück, etwa mittels Laser, herausgeschnitten sind.

4. Stent nach Anspruch 1 oder 2, **dadurch gekennzeichnet, daß** die beiden die gegenläufigen Wendeln (3, 3') bildenden Filamente (2, 2') an einer zweiten Verbindungsstelle (5) miteinander verklebt, verlötet oder verschweißt sind.

5. Stent nach Ansprüche 1 oder 2, **dadurch gekennzeichnet, daß** die beiden die gegenläufigen Wendeln (3, 3') bildenden Filamente (2, 2') an einer zweiten Verbindungsstelle (5) mittels einer die beiden Filamente (2, 2') übergreifenden Muffe (5, 27) miteinander verbunden sind.

6. Stent nach einem der vorhergehenden Ansprüche, **dadurch gekennzeichnet, daß** die gegenläufigen Wendeln (3, 3') zumindest teilweise in ihren Überkreuzungspunkten (24) miteinander verbunden sind.

7. Stent nach Anspruch 6, **dadurch gekennzeichnet, daß** die Verbindung der gegenläufigen Wendeln (3, 3') an den Überkreuzungspunkten (24) mittels Schlaufen von an wenigstens einem der Filamente (2, 2') angeordneten Fäden (25) hergestellt ist, wobei die Filamente (2, 2') gegeneinander begrenzt beweglich sind.

8. Stent nach einem der vorhergehenden Ansprüche, **gekennzeichnet durch** wenigstens drei Filamente (2, 2', 12) annähernd gleicher Länge mit einer bestimmungsgemäßen Wendelform, wobei das dritte bzw. die folgenden Filamente (12) in seiner/ihrer bestimmungsgemäßen Form eine zumindest teilweise zu den beiden die gegenläufigen Wendeln (3, 3') bildenden Filamenten (2, 2') unterschiedliche Steigung aufweist/aufweisen.

9. Stent nach einem der vorhergehenden Ansprüche, **dadurch gekennzeichnet, daß** die Filamente (2, 2') über wenigstens einen Teil der Gesamtlänge des Stents (1) den gleichen Drehsinn aufweisen.

10. Stent nach einem der vorhergehenden Ansprüche, **dadurch gekennzeichnet, daß** wenigstens ein Filament (2, 2') in seinem bestimmungsgemäßen Zustand einen entlang dem Außenumfang des Stents (1, 10) verlaufenden, bogenförmigen Abschnitt (29, 29') aufweist.

11. Stent nach Anspruch 10, **dadurch gekennzeichnet, daß** jeweils zwei Filamente (2, 2') wenigstens über einen Teil der Längenerstreckung des Stents (1, 10) jeweils gleichartige, zueinander gegenläufige bogenförmige Abschnitte (29, 29') aufweisen.

12. Stent nach Anspruch 11, **dadurch gekennzeichnet, daß** die bogenförmigen Abschnitte im Bereich ihrer maximalen Annäherung fest, etwa mittels einer die Filamente (2, 2') verbindenden Muffe und/oder durch verschweißen, Verlöten oder Verkleben, oder derart miteinander verbunden sind, daß eine begrenzte Beweglichkeit der Filamente (2, 2') gegeneinander gewahrt bleibt, etwa mittels Schlaufen von an wenigstens einem der Filamente (2, 2') angeordneten Fäden (25).

13. Stent nach Anspruch 12, **dadurch gekennzeichnet, daß** zwei der Filamente (2, 2') in ihrer bestimmungsgemäßen Form jeweils eine alternierende Struktur aufweisen, bei der wenigstens eine vollständige Wendelschlaufe (30, 30') auf wenigstens einen bogenförmigen Abschnitt (29, 29') folgt, und die Filamente (2, 2') dabei gegeneinander derart versetzt angeordnet sind, daß jeweils eine Wendelschlaufe (30, 30') des einen Filaments (2) einen bogenförmigen Abschnitt (29, 29') des anderen Filaments (2') überkreuzt.

14. Stent nach einem der vorhergehenden Ansprüche, **dadurch gekennzeichnet, daß** der Stent (1, 10) zumindest abschnittsweise ein ovales oder "D"-förmiges Lumen aufweist.

15. Stent nach einem der vorhergehenden Ansprüche, **dadurch gekennzeichnet, daß** die Filamente (2, 2') in ihrer bestimmungsgemäßen Form über wenigstens einen Teil der Längserstreckung des Stents (1) einen aus wenigstens zwei Nebenstents (61, 61') bestehenden Doppelstent bilden.

16. Stent nach Anspruch 16, **dadurch gekennzeichnet, daß** die Nebenstents (61, 61') des Doppelstents an den Wendelschlaufen (30, 30') ihrer Filamente (2, 2') miteinander verbunden sind.

17. Stent nach Anspruch 16, **dadurch gekennzeichnet, daß** der Stent (1) in seinem bestimmungsgemäßen Zustand wenigstens an seinem distalen Ende derart geformt ist, daß das Lumen des Stents (1) die ungefähre Form einer "Acht" aufweist.

18. Stent nach einem der vorhergehenden Ansprüche, **gekennzeichnet durch** eine vorzugsweise elastisch verformbare Membranumhüllung (66).

19. Stent nach Anspruch 18, **dadurch gekennzeichnet, daß** die Membranumhüllung (66) aus einem Textilgewebe besteht.

20. Stent nach Anspruch 21, **dadurch gekennzeichnet, daß** in die Gewebestruktur der textilen Membranumhüllung (66) Verstärkungsfäden (59), vorzugsweise aus Metall, eingewirkt sind.

21. Stent nach einem der Ansprüche 1 bis 20, **dadurch gekennzeichnet, daß** wenigstens eines der Filamente (12, 2') in seiner bestimmungsgemäßen Ausbildung zumindest teilweise mit einer über den Außenumfang dieses Filaments hinausragenden Gewebestruktur (45) und/oder mit sich radial vom Filament forterstreckenden Fasern (48, 49) versehen sind.

22. Stent nach Anspruch 21, **dadurch gekennzeichnet, daß** sich die von den einzelnen, benachbarten, von den Filamenten (2, 2') gebildeten Wendeln (46, 47) und/oder Bogenabschnitten (29, 29') ausgehenden Gewebestrukturen (45) und/oder Fasern (48, 49) an ihren freien Enden zumindest teilweise berühren.

23. Stent nach Anspruch 21 oder 22, **dadurch gekennzeichnet, daß** wenigstens ein Filament (2, 2') unter Einschluß von überstehenden Gewebestrukturen (45) und/oder radial abstehenden Fasern (48, 49) umhüllt und/oder umwickelt ist.

24. Stent nach Anspruch 23, **dadurch gekennzeichnet, daß** mehrere Teilfilamente (55, 55') unter Einschluss von überstehenden Gewebestrukturen (45) und/oder radial abstehenden Fasern (48, 49) miteinander zu einem einzigen Filament (2, 2') verdrillt sind.

25. Stent nach einem der Ansprüche 21 bis 24, **dadurch gekennzeichnet, daß** die sich vom Filament (2, 2') radial forterstreckenden Gewebestrukturen (45) und/oder Fasern (48, 49) abschnittsweise und/oder je nach radialer Vorzugsrichtung von unterschiedlicher Länge sind.

26. Stent nach Anspruch 25, **dadurch gekennzeichnet, daß** sich die Gewebestrukturen (45) und/oder die Fasern (48, 49) zwischen den einzelnen, benachbarten, von den Filamenten (2, 2') gebildeten Wendelschlaufen (46, 47) und/oder Bogenabschnitten (29, 29') unter Ausbildung einer wellenlinienförmigen Begrenzungslinie (51) zumindest annähernd berühren.

27. Stent nach einem der Ansprüche 21 bis 26, **dadurch gekennzeichnet, daß** sich die Gewebestrukturen (45) und/oder die Fasern (48, 49) zwischen den einzelnen, benachbarten, von den Filamenten (2, 2') gebildeten Wendelschlaufen (46, 47) und/oder Bogenabschnitten (29, 29') zumindest teilweise überlappen.

28. Stent nach einem der Ansprüche 21 bis 27, **dadurch gekennzeichnet, daß** die Gewebestruktur (45) zumindest teilweise aus einer Textilstruktur besteht.

29. Stent nach einem der Ansprüche 21 bis 27, **dadurch gekennzeichnet, daß** die Gewebestruktur (45) zumindest teilweise aus Metallgewebe hergestellt ist.

30. Stent nach einem der Ansprüche 28 oder 29, **dadurch gekennzeichnet, daß** die Gewebestruktur (45) zumindest abschnittsweise fransenartig eingeschnitten ist.

31. Stent nach einem der Ansprüche 28 bis 30, **dadurch gekennzeichnet, daß** die Gewebestruktur (45) mit einem Haftmittel, vorzugsweise einem Klettverschluß (64) zur Verbindung der einander überlappenden Gewebestrukuren versehen ist.

32. Stent nach einem der Ansprüche 28 bis 31, **dadurch gekennzeichnet, daß** der Querschnitt der Gewebestruktur (45) derart ausgebildet ist, daß er sich mit zunehmendem Abstand von dem Filament (2, 2') verjüngt.

33. Stent nach einem der vorhergehenden Ansprüche, **dadurch gekennzeichnet, daß** ein Dichtungsstent (67, 68, 69) Gewebestrukturen (45) und/oder Fasern (48, 49) aufweist, die bevorzugt radial in das Lumen des Stents (1, 10) hinein- und/oder radial über den Außenumfang des Stents (1, 10) hinausragen.

34. Stent nach einem der vorhergehenden Ansprüche, **dadurch gekennzeichnet, daß** wenigstens eines der Filamente (2, 2') mit Markierungen versehen ist, die eine externe Beobachtung des Stents (1) mit diagnostischen Mitteln, etwa mit Röntgendiagnostik, erlauben.

35. Stent nach einem der vorhergehenden Ansprüche, **dadurch gekennzeichnet, daß** die Filamente (2, 2') in vorbestimmten Abständen entlang der Längserstreckung des Stents (1) jeweils zueinander entgegengesetzt gewendelte, den Außenradius des Stents bestimmende Schlaufen (66) ausbilden.

## Claims

1. Stent for treatment of pathological body vessels, which can be implanted permanently and adopts its intended form only at the implantation location during the implantation, and comprises several filaments, wherein the filaments (2, 2') in the intended state of the stent (1) are fixedly connected together at at least one connecting point (4, 5) lying in the region of the outer circumference of the stent (1), wherein at least two of the filaments (2, 2') in their intended state are, over at least a part of the length of the stent (1), present in the form of helices (3, 3') of mutually opposite sense and these filaments (2, 2') of the helices (3, 3') of mutually opposite sense consist of a single filament wire, which at a first of the at least one connecting point of the two helices (3, 3') has a kink or a loop (4) for formation of a helix (3, 3') of opposite sense, **characterised in that** the stent (1) is introducible in the form of at least two longitudinally extended filaments (2, 2') into a body vessel (21, 60) by means of an implantation device (70) and the first of the at least one connecting point of the two helices is respectively arranged at one of the front ends of the stent (1).

2. Stent according to claim 1, **characterised in that** the filaments (2, 2') consist of a material with superelastic characteristics and/or a thermo-memory characteristic.

3. Stent according to claim 1 or 2, **characterised in that** the filaments (2, 2') of the helices (3, 3') of opposite sense are cut out integrally from a tubular workpiece, for example by means of laser.

4. Stent according claim 1 or 2, **characterised in that** the two filaments (2, 2') forming the helices (3, 3') of opposite sense are glued, soldered or welded together at a second connecting point (5).

5. Stent according to claim 1 or 2, **characterised in that** the two filaments (2, 2') forming the helices (3, 3') of opposite sense are connected together at a second connecting point (5) by means of a sleeve (5, 27) engaging over the two filaments (2, 2').

6. Stent according to one of the preceding claims, **characterised in that** the helices (3, 3') of opposite sense are connected together at least partly at the points (24) of intersection thereof.

7. Stent according to claim 6, **characterised in that** the connection of the helices (3, 3') of opposite sense at the points (24) of intersection is produced by means of loops of threads (25) arranged at at least one of the filaments (2, 2'), wherein the filaments (2, 2') are capable of limited movement relative to one another.

8. Stent according to one of the preceding claims, **characterised by** at least three filaments (2, 2', 12) of approximately equal length with an intended helical form, wherein the third filament or the following filaments (12) has or have in the intended form thereof a slope differing at least partly from the two filaments (2, 2') forming the helices (3, 3') of opposite sense.

9. Stent according to one of the preceding claims, **characterised in that** the filaments (2, 2') have the same sense of rotation over at least a part of the total length of the stent (1).

10. Stent according to one of the preceding claims, **characterised in that** at least one filament (2, 2') has in its intended state a curved section (29, 29') extending along the outer circumference of the stent (1, 10).

11. Stent according to claim 10, **characterised in that** each time two filaments (2, 2') have over a part of the length of the stent (1, 10) each time identical curved sections (29, 29') of mutually opposite sense.

12. Stent according to claim 11, **characterised in that** the curved sections in the region of their maximum approach are fixedly connected together for example by means of a sleeve connecting the filaments (2, 2') and/or by welding, soldering or gluing or are connected together in such a manner that a limited capability of movement of the filaments (2, 2') relative to one another remains ensured, for example by means of loops of threads (25) arranged at at least one of the filaments (2, 2').

13. Stent according to claim 12, **characterised in that** two of the filaments (2, 2') in their intended form each time have an alternating structure in which at least one complete helical loop (30, 30') follows at least one curved section (29, 29') and the filaments (2, 2') **in that** case are arranged offset relative to one another in such a manner that each time one helical loop (30, 30') of one filament (2, 2') intersects a curved section (29, 29') of the other filament (2, 2').

14. Stent according to one of the preceding claims, **characterised in that** the stent (1, 10) has an oval or a 'D'-shaped lumen at least in sections.

15. Stent according to one of the preceding claims, **characterised in that** the filaments (2, 2'), in their intended form, form over at least a part of the length of the stent (1) a double stent consisting of at least two adjacent stents (61, 61').

16. Stent according to claim 15, **characterised in that** the adjacent stents (61, 61') of the double stent are connected together at the helical loops (30. 30') of their filaments (2, 2').

17. Stent according to claim 16, **characterised in that** the stent (1) in its intended state is formed at at least its distal end in such a manner that the lumen of the stent (1) has the approximate shape of an 'eight'.

18. Stent according to one of the preceding claims, **characterised by** a preferably resiliently deformable membrane enclosure (66).

19. Stent according to claim 18, **characterised in that** the membrane enclosure (66) consists of a textile fabric.

20. Stent according to claim 21, **characterised in that** reinforcing threads (59), preferably of metal, are worked into the fabric structure of the textile membrane enclosure (66).

21. Stent according to one of claims 1 to 20, **characterised in that** at least one of the filaments (12, 2') in its intended construction is provided at least partly with a fabric structure (45), which projects beyond the outer circumference of this filament, and/or with fibres (48, 49) extending radially from the filament.

22. Stent according to claim 21, **characterised in that** the fabric structures (45) and/or fibres (48, 49), which go out from the individual adjacent helices (46, 47) and/or curve sections (29, 29') formed by the filaments (2, 2'), at least partly contact one another at their free ends.

23. Stent according to claim 21 or 22, **characterised in that** at least one filament (2, 2') is enclosed and/or wound around with inclusion of fabric structures (45) protruding beyond and/or fibres (48, 49) radially protruding away.

24. Stent according to claim 23, **characterised in that** several part filaments (55, 55') are twisted together, with inclusion of fabric structures (45) protruding beyond and/or fibres (48, 49) radially protruding away, to form a single filament (2, 2').

25. Stent according to one of claims 21 to 24, **characterised in that** the fabric structures (45) and/or fibres (48, 49) radially extending from the filament (2, 2') are of different length in sections and/or according to the respective radial preferential direction.

26. Stent according to claim 25, **characterised in that** the fabric structures (45) and/or the fibres (48, 49) between the individual adjacent helical loops (46, 47) and/or curved sections (29, 29') formed by the filaments (2, 2') at least approximately contact one another with formation of a sinusoidal boundary line (51).

27. Stent according to one of claims 21 to 26, **characterised in that** the fabric structures (45) and/or the fibres (48, 49) at least partly overlap between individual adjacent helical loops (46, 47) and/or curved sections (29, 29') formed by the filaments (2, 2').

28. Stent according to one of claims 21 to 27, **characterised in that** the fabric structure (45) is made at least partly of a textile structure.

29. Stent according to one of claims 21 to 27, **characterised in that** the fabric structure (45) is made at least partly of metal fabric.

30. Stent according to one of claims 28 or 29, **characterised in that** the fabric structure (45) is incised at least in sections in fringe-like manner.

31. Stent according to one of claims 28 to 30, **characterised in that** the fabric structure (45) is provided with adhering means, preferably a hook-and-burr fastener (64), for connecting the mutually overlapping fabric structures.

32. Stent according to one of claims 28 to 31, **characterised in that** the cross-section of the fabric structure (45) is formed in such a manner that it tapers with increasing spacing from the filament (2, 2').

33. Stent according to one of the preceding claims, **characterised in that** a sealing stent (67, 68, 69) comprises fabric structures (45) and or fibres (48, 49) which preferably protrude radially into the lumen of the stent (1, 10) and/or protrude radially out beyond the outer circumference of the stent (1, 10).

34. Stent according to one of the preceding claims, **characterised in that** at least one of the filaments (2, 2') is provided with markings which permit external observation of the stent (1) by diagnostic means, for example by X-ray diagnostic equipment.

35. Stent according to one of the preceding claims, **characterised in that** the filaments (2, 2') form, at predetermined spacings along the length of the stent (1), respective mutually oppositely coiled loops (66) determining the outer radius of the stent.

## Revendications

1. Extenseur pour le traitement de vaisseaux corporels pathologiques, qui peut être implanté d'une manière durable et qui prend seulement à l'emplacement d'implantation, pendant l'implantation, sa forme prévue, et comprend plusieurs filaments, où les filaments (2,2'), à l'état prévu de l'extenseur (1), sont reliés solidement les uns aux autres à au moins un emplacement de liaison (4,5) situé au voisinage du pourtour extérieur de l'extenseur (1), où au moins deux des filaments (2, 2'), dans leur état prévu, font saillie sur au moins une partie de l'extension longitudinale de l'extenseur (1) sous la forme d'hélices opposées (3,3'), et ces filaments (2,2') des hélices (3,3')à mouvement opposé sont constitués d'un seul fil de filament qui présente à un premier d'au moins un emplacement de liaison des deux hélices (3,3') un pli ou une boucle (4) pour former une hélice opposée (3,3'), **caractérisé en ce que** l'extenseur (1) est insérable sous la forme d'au moins deux filaments oblongs (2,2') au moyen d'un dispositif d'implantation (70) dans un vaisseau corporel (21,60) et **en ce que** le premier d'au moins un emplacement de liaison des deux hélices est disposé à chaque fois à l'une des extrémités frontale de l'extenseur (1).

2. Extenseur selon la revendication 1, **caractérisé en ce que** les filaments (2,2') sont constitués d'un matériau ayant des propriétés super-élastiques et/ou une propriété de mémoire thermique.

3. Extenseur selon la revendication 1 ou 2, **caractérisé en ce que** les filaments (2,2') des hélices opposées (3,3') sont découpés en une pièce d'une pièce tubulaire, par exemple au moyen d'un laser.

4. Extenseur selon la revendication 1 ou 2, **caractérisé en ce que** les deux filaments (2,2') formant les hélices opposées (3, 3') sont assemblés par collage, par brasage, ou par soudage à un deuxième emplacement de liaison (5).

5. Extenseur selon la revendication 1 ou 2, **caractérisé en ce que** les deux filaments (2,2') formant les hélices opposées (3,3') sont reliés à un deuxième emplacement de liaison (5) au moyen d'un manchon (5,27) passant sur les deux filaments (2,2').

6. Extenseur selon l'une des revendications précédentes, **caractérisé en ce que** les hélices opposées (3,3') sont reliées au moins partiellement à leurs points de croisement (24).

7. Extenseur selon la revendication 6, **caractérisé en ce que** la liaison des hélices opposées (3, 3') , aux points de croisement (24), est réalisée au moyen de boucles de fils (25) disposés à au moins l'un des filaments (2,2'), où les filaments (2,2') sont déplaçables d'une manière limitée l'un contre l'autre.

8. Extenseur selon l'une des revendications précédentes, **caractérisé par** au moins trois filaments (2,2',12) d'une longueur approximativement égale avec une forme d'hélice prévue, où le troisième respectivement les filaments suivants (12) présentent dans leur forme prévue un pas au moins partiellement différent par rapport aux deux filaments (2,2') formant les hélices opposées (3, 3').

9. Extenseur selon l'une des revendications précédentes, **caractérisé en ce que** les filaments (2,2') présentent sur au moins une partie de la longueur totale de l'extenseur (1) le même sens de rotation.

10. Extenseur selon l'une des revendications précédentes, **caractérisé en ce qu'**au moins un filament (2,2') présente dans son état prévu un tronçon courbé (29,29') s'étendant le long du pourtour extérieur de l'extenseur (1, 10).

11. Extenseur selon la revendication 10, **caractérisé en ce que** respectivement deux filaments (2,2') présentent au moins sur une partie de l'extension longitudinale de l'extenseur (1,10) respectivement des tronçons arqués (29,29')opposés, du même type.

12. Extenseur selon la revendication 11, **caractérisé en ce que** les tronçons arqués sont reliés au voisinage de leur rapprochement maximal solidement, par exemple au moyen d'un manchon reliant les filaments (2,2') et/ou par soudage, brasage ou collage de telle sorte qu'un déplacement limité des filaments (2,2') l'un contre l'autre reste assuré, par exemple au moyen de boucles de fils (25) disposés à au moins l'un des filaments (2, 2').

13. Extenseur selon la revendication 12, **caractérisé en ce que** deux des filaments (2,2'), dans leur forme prévue, ont chacun une structure alternante, où au moins une boucle d'hélice complète (30,30') fait suite à au moins un tronçon arqué (29,29'), et les filaments (2,2') sont dans ce cas décalés l'un relativement à l'autre de façon que respectivement une boucle d'hélice (30,30') d'un filament (2) croise un tronçon arqué (29,29') de l'autre filament (2').

14. Extenseur selon l'une des revendications précédentes, **caractérisé en ce que** l'extenseur (1,10) présente au moins par tronçons une lumière ovale ou en forme de "D".

15. Extenseur selon l'une des revendications précédentes, **caractérisé en ce que** les filaments (2,2'), dans leur forme prévue, forment sur au moins une partie de l'extension longitudinale de l'extenseur (1) un extenseur double constitué d'au moins deux extenseurs secondaires (61,61').

16. Extenseur selon la revendication 15, **caractérisé en ce que** les extenseurs secondaires (61,61') de l'extenseur double sont reliés entre eux aux boucles d'hélice (30, 30') de leurs filaments (2,2').

17. Extenseur selon la revendication 16, **caractérisé en ce que** l'extenseur (1), dans son état prévu, est formé au moins à son extrémité distale de façon que la lumière de l'extenseur (1) présente la forme approximative d'un "huit".

18. Extenseur selon l'une des revendications précédentes, **caractérisé par** une enveloppe de membrane (66) de préférence déformable élastiquement.

19. Extenseur selon la revendication 18, **caractérisé en ce que** l'enveloppe de membrane (66) est constituée d'un tissu textile.

20. Extenseur selon la revendication 21, **caractérisé en ce que** sont tricotés dans la structure de tissu de l'enveloppe de membrane textile (66) des fils de renforcement (59), de préférence en métal.

21. Extenseur selon l'une des revendications 1 à 20, **caractérisé en ce qu'**au moins l'un des. filaments (2,2'), dans sa réalisation prévue, est pourvu au moins partiellement d'une structure de tissu (45) faisant saillie sur le pourtour extérieur de ce filament et /ou de fibres (48,49) s'étendant radialement au loin du filament.

22. Extenseur selon la revendication 21, **caractérisé en ce que** les structures de tissu (45) et/ou fibres (48,49) s'étendant à partir des boucles individuelles avoisinantes (46,47) formées par les filaments (2, 2') et/ou tronçons arqués (29, 29'), et/ou fibres (48,49) se touchent au moins partiellement à leurs extrémités libres.

23. Extenseur selon la revendication 21 ou 22, **caractérisé en ce qu'**au moins un filament (2,2'), par l'inclusion de structures de tissu saillantes (45) et/ou fibres dépassant radialement (48,49), est enveloppé et/ou enroulé.

24. Extenseur selon la revendication 23, **caractérisé en ce que** plusieurs filaments partiels (55, 55'), sous l'inclusion de structures de tissu saillantes (45) et/ou fibres dépassant radialement (48,49) sont torsadés ensemble en un seul filament (2,2').

25. Extenseur selon l'une des revendications 21 à 24, **caractérisé en ce que** les structures de tissu (45) et/ou fibres (48,49) s'étendant radialement au loin du filament (2,2') sont d'une longueur différente par tronçons et/ou selon la direction de traction radiale.

26. Extenseur selon la revendication 25, **caractérisé en ce que** les structures de tissu (45) et /ou les fibres (48,49) entre les boucles d'hélice individuelles avoisinantes (46,47) formées par les filaments (2,2') et/ou les tronçons arqués (29,29'), en réalisant une ligne de délimitation (51) en forme de ligne d'onde, se touchent au moins approximativement.

27. Extenseur selon l'une des revendications 21 à 26, **caractérisé en ce que** les structures de tissu (45) et/ou les fibres (48,49) entre les boucles d'hélice individuelles avoisinantes (46, 47) formées par les filaments (2,2') et/ou les tronçons arqués (29,29') se chevauchent au moins partiellement.

28. Extenseur selon l'une des revendications 21 à 27, **caractérisé en ce que** la structure de tissu (45) est constituée au moins partiellement d'une structure de textile.

29. Extenseur selon l'une des revendications 21 à 27, **caractérisé en ce que** la structure de tissu (45) est réalisée au moins partiellement en tissu métallique.

30. Extenseur selon l'une des revendications 28 ou 29, **caractérisé en ce que** la structure de tissu (45) est entaillée au moins par sections à la manière d'une frange.

31. Extenseur selon l'une des revendications 28 à 30, **caractérisé en ce que** la structure de tissu (45) est pourvue d'un moyen d'adhésion, de préférence d'une fermeture à auto-accrochage (64) pour assembler les structures de tissu qui se chevauchent.

32. Extenseur selon l'une des revendications 28 à 31, **caractérisé en ce que** la section transversale de la structure de tissu (45) est réalisée de façon à diminuer au fur et à mesure qu'augmente l'écart du filament (2, 2').

33. Extenseur selon l'une des revendications précédentes, **caractérisé en ce qu'**un extenseur d'étanchéité (67,68,69) présente des structures de tissu (45) et/ou des fibres (48,49) qui font saillie de préférence radialement dans la lumière de l'extenseur (1,10) et/ou radialement sur le pourtour extérieur de l'extenseur (1,10).

34. Extenseur selon l'une des revendications précédentes, **caractérisé en ce qu'**au moins l'un des filaments (2,2') présente des marquages qui permettent une observation externe de l'extenseur (1) avec des moyens diagnostiques, par exemple avec un diagnostique à rayons X.

35. Extenseur selon l'une des revendications précédentes, **caractérisé en ce que** les filaments (2,2'), à des écarts prédéterminés le long de l'extension longitudinale de l'extenseur (1), forment des boucles (66) à enroulement opposé, déterminant le rayon extérieur de l'extenseur.
